# EUROPEAN PATENT APPLICATION

(11) **EP 2 455 081 A1**
(43) Date of publication of application: **23.05.2012**
(21) Application number: 10382313.4
(22) Date of filing: 23.11.2010
(51) Int. Cl.: A61K 31/4245, A61P 1/04

(54) **S1P1 receptor agonists for use in the treatment of crohn's disease**

(71) Applicant: Almirall, S.A., 08022 Barcelona (ES)
(72) Inventor: Godessart Marina, Nuria, 08980 San Feliu de LLobregat, Barcelona (ES); Tarrason Encuentra, Gema, 08980 Sant Feliu de Llobregat, Barcelon (ES); Aguilar Izquierdo, Nuria, 08980 San Feliu de Llobregat, Barcelona (ES)
(74) Representative: Elzaburu Marquez, Alberto

(57) **Abstract**

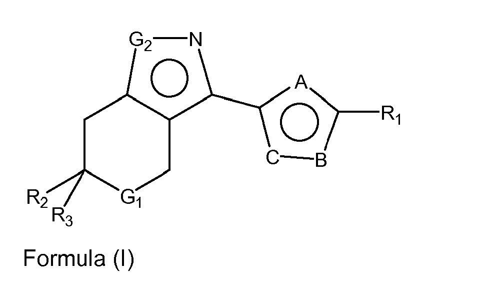

a S1P1 receptor agonist of formula (I), or a pharmaceutically acceptable salt, or solvate, or N-oxide, or stereoisomer or deuterated derivate thereof, for use in the treatment of inflammatory bowel disease, in particular Crohn's disease.

## Description

The present invention relates to S1P1 agonist derivatives of formula (I) for use in the treatment of inflammatory bowel disease, in particular Crohn's disease.

### BACKGROUND OF THE INVENTION

Inflammatory bowel diseases (IBDs) are chronic, relapsing and tissue-destructive diseases of the gastrointestinal tract characterized by a dysfunction of mucosal T cells, imbalanced cytokine production and cellular inflammation leading to damage of the intestinal mucosa. The major forms of idiopathic IBD are ulcerative colitis and Crohn's disease, and they are distinguished by specific clinical, pathological, endoscopic and radiological features. IBD is the result of a combination of both environmental and host factors, which vary depending genetic inheritance at several susceptibility loci. Genetic factors discovered to date affect epithelial barrier function, as well as innate and adaptive immunity. Accumulating evidence suggest that the dynamic balance between microbe, specially commensal flora, and host defensive responses at the mucosal frontier has a pivotal role in the initiation and pathogenesis of chronic IBD.

The main difference between Crohn's disease and ulcerative colitis is the location and nature of the inflammatory changes. Crohn's disease can affect any part of the gastrointestinal tract, from mouth to anus, although a majority of the cases start in the terminal ileum. Ulcerative colitis, in contrast, is restricted to the colon and the rectum. Unlike ulcerative colitis, Crohn's disease may be patchy and segmental, and inflammation is tipically transmural (R. J. Xavier and D. K. Podolsky, "Unravelling the pathogenesis of inflammatory bowel disease". Nature, 2007, 448: 427-434).

The organized lymphoid tissue of the gastrointestinal tract contains large numbers of immune cells that are deputed both to protect from infectious diseases and to evoke immune tolerance. Perturbations in this delicately balanced microenvironment can promote the collapse of tolerance, thus leading to chronic inflammation that alters the integrity and function of the gut. In both, Crohn's disease and ulcerative colitis, the tissue damage is mediated by an excessive and poorly controlled immune-inflammatory reaction directed against components of the normal bacterial flora. Evidence also indicates that an active and dynamic interplay between immune and non-immune cells plays a major role in initiating and shaping this pathologic process, and that cytokines are essential mediators of this cross-talk.

Current treatments of IBD comprise biological agents such as infliximab, immunosuppressants, such as azathioprine or 6-mercapthopurine, and anti-inflammatory agents, such as sulphasalazine. Corticosteroids are usually administered during relapses.

### S1P1 agonists and experimental colitis

Sphingosine-1-phosphate receptor agonists are a new class of immunomodulators that inhibit the egress of T cells from lymph nodes, thereby preventing pathogenic T cells from migrating towards target tissues in chronic immune-mediated diseases.

Fingolimod, an orally active S1P1 receptor agonist, has recently been approved by the FDA for the treatment of Multiple Sclerosis. It is presumed that fingolimod acts by preventing the infiltration of the CNS by autoreactive lymphocytes and the subsequent destruction of the myelin sheath characteristic of this disease.

At present, there are no S1P1 receptor agonists in the clinic for the treatment of IBD. However, based on their mechanism, it is likely that these compounds may have a therapeutic utility on these disorders. In fact, preclinical data has shown that fingolimod improves the intestinal inflammation that takes place in experimental models of colitis in rodents.

In two mice models of colitis; the dextran sulphate sodium -induced colitis as well as in the CD4+CD26L+ T cell transfer model, fingolimod has been reported to improve the colitis activity index and to diminish mucosal edema, cellular infiltration and epithelial disruption. In both models, fingolimod prevented the infiltration of CD4+ T cells into the inflamed colonic lamina propria (Deguchi Y et al. 2006. The S1P receptor modulator FTY720 prevents the development of experimental colitis in mice. Oncol. Rep. 16(4): 699-703.

Thus there is need to provide new S1 P1 receptor agonists useful for the treatment of inflammatory bowel disease, in particular Crohn's disease.

### DESCRIPTION OF THE INVENTION

It has been found that the S1 P1 agonists of the present invention do not cause hepatotoxicity in vivo, have a shorter half-life in plasma and a faster recovery of lymphopenia, when compared with Fingolimod. These features render these compounds potentially safer as a therapy for Crohn's disease patients.

Thus, the present invention is directed to a S1 P1 receptor agonist of formula (I) or a pharmaceutically acceptable salt, or solvate, or N-oxide, or stereoisomer or deuterated thereof, for use in the treatment of Crohn's disease: 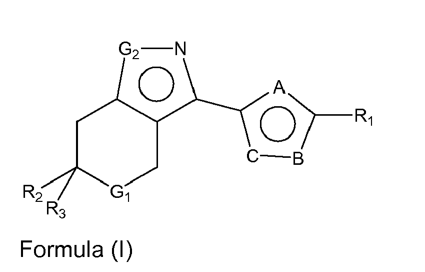 wherein,
either (i) A is selected from the group consisting of -N-, -O- and -S-; B and C are independently selected from the group consisting of -N- and -O-, with the proviso that two of A, B and C are nitrogen atoms, or (ii) two of A, B and C are -N- and one of A, B and C is -NH-;
G¹ is selected from the group consisting of -CH₂-, -NH- and -O-;
G² is selected from the group consisting of -NR⁴- and -O-;
R¹ represents:
➢ a 8 to 10 membered bicyclic N-containing heteroaryl group optionally substituted with a C₁₋₄ carboxyalkyl group or a C₁₋₄ aminoalkyl group,
➢ a pyridyl group optionally substituted with one or more substituents selected from hydroxy groups, C₁₋₄ alkyl groups, C₁₋₄ carboxyalkyl groups, C₁₋₄ haloalkyl groups, C₁₋₄ alkoxy groups, amino groups, C₁₋₄ aminoalkyl groups and C₁₋₄ aminoalkoxy groups,
➢ a pyridone group substituted with one or more C₁₋₄ alkyl groups; C₁₋₄ haloalkyl groups or C₁₋₄ aminoalkyl groups, or
➢ a group of formula: 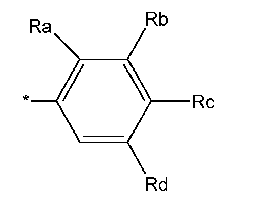 wherein:
   - R^{a} represents a hydrogen atom, a halogen atom, a C₁₋₄ alkyl group, C₃₋₄ cycloalkyl group or a -CF₃ group;
   - R^{b} represents a hydrogen atom, a halogen atom, a C₁₋₄ alkyl group, a -CF₃ group or a C₁₋₄ alkoxy group;
   - R^{d} represents a hydrogen atom, a C₁₋₄ alkyl group or a C₁₋₄ alkoxy group;
   - R^{c} represents:
      o a hydrogen atom, a C₁₋₄ hydroxyalkyl group, a C₁₋₄ aminoalkyl group which is optionally substituted with one or more substituents selected from halogen atoms, hydroxy groups and -CF₃ groups;
      o a 4 to 6-membered saturated N-containing heterocyclic ring optionally substituted with a C₁₋₂ carboxyalkyl group;
      o -(CH₂)₍₀₋₄₎-C(O)OR', -(CH₂)₍₀₋₄₎-C(O)NR'R", -(CH₂)₍₀₋₄₎-NHC(O)R", -S(O)₂NR'R", -O-(CH₂)₍₂₋₄₎NR'R", -O-(CH₂)₍₁₋₄₎C(O)OR", -O-(CH₂)₍₁₋₄₎-C(O)NR'R", -(CH₂)₍₀₋₄₎-NR'R", -(CH₂)₍₀₋₄₎-CONHS(O)₂R', -(CH₂)₍₀₋₄₎-NHS(O)₂R' or -(CH₂)₍₀₋₃₎-NH-(CH₂)₍₁₋₃₎-(NH)₍₀₋₁₎S(O)₂R' wherein,
         ■ R' represents a hydrogen atom or a C₁₋₄ alkyl group,
         ■ R" represents a hydrogen atom, a C₁₋₄ alkyl group, a C₃₋₄ cycloalkyl group, a C₁₋₄ carboxyalkyl group, a C₁₋₄ haloalkyl group, a C₁₋₄ hydroxyalkyl group or a 6 membered, saturated N-containing heterocyclic ring, or
         ■ R' and R" together with the nitrogen atom to which they are attached form a 4 to 6 membered heterocyclic group which contains, as heteroatoms, one N atom and, optionally, one further atom selected from N and O and which is optionally substituted with a carboxy or a C₁₋₄ carboxyalkyl group"
      or R^{c} together with R^{d} form a C₅₋₆ cycloalkyl group optionally substituted by a -NHR^{f} group, wherein R^{f} is selected from the group consisting of a hydrogen atom and a carboxymethyl group;

R² and R³ are independently selected from the group consisting of hydrogen atoms, halogen atoms and C₁₋₄ alkyl groups; and

R⁴ is selected from the group consisting of a hydrogen atom, a phenyl group, a C₃₋₄ cycloalkyl-C₁₋₄ alkyl group, C₁₋₄ aminoalkyl group, C₁₋₄ haloalkyl group and a linear or branched C₁₋₄ alkyl group which is optionally substituted by a phenyl or a pyridyl group.

The S1 P1 receptor agonists of formula (I) and methods for their preparation are described in International Patent Application PCT/EP2009/008968, published as WO2010/072352

As used herein the term alkyl embraces optionally substituted, linear or branched hydrocarbon radicals having 1 to 8, preferably 1 to 4 carbon atoms. Examples include methyl, ethyl, *n*-propyl, *i*-propyl, *n*-butyl and *tert*-butyl radicals.

As used herein, a haloalkyl group is a said alkyl group, for example a C₁₋₄ or C₁₋₂ alkyl group, which is attached to 1, 2 or 3 halogen atoms. The halogen atom is preferably a fluorine atom. Preferably, said haloalkyl group is chosen from -CH₂F -CF₂H, -CF₃ and -CH₂CF₃. -CF₃ and -CH₂CF₃ are preferred.

As used herein, the term hydroxyalkyl embraces linear or branched alkyl radicals having 1 to 4 carbon atoms, any one of which may be substituted with one or more hydroxyl radicals. Examples of such radicals include hydroxymethyl, hydroxyethyl, hydroxypropyl, hydroxybutyl and 1,2-dihydroxypropyl.

As used herein, the term aminoalkyl embraces linear or branched alkyl radicals having 1 to 4 carbon atoms, any one of which may be substituted with one or more amino groups. Examples of such radicals include aminomethyl, aminoethyl, aminopropyl and aminobutyl.

As used herein, the term carboxyalkyl embraces linear or branched alkyl radicals having 1 to 4 carbon atoms, any one of which may be substituted with one or more carboxy radicals. Examples of such radicals include carboxymethyl, carboxyethyl, carboxypropyl, carboxybutyl and 1,2-dicarboxypropyl.

As used herein the term alkoxy embraces optionally substituted, linear or branched oxy-containing radicals each having 1 to 8, preferably, 1 to 4 carbon atoms. Examples include methoxy, ethoxy, *n*-propoxy, *i*-propoxy, *n*-butoxy and *tert*-butoxy radicals.

As used herein, the term aminoalkoxy embraces linear or branched alkoxy radicals having 1 to 4 carbon atoms, any one of which may be substituted with one or more amino groups. Examples of such radicals include aminomethoxy, aminoethoxy, aminopropoxy and aminobutoxy.

As used herein, the term cycloalkyl embraces optionally substituted saturated carbocyclic radicals and, unless otherwise specified, a cycloalkyl radical typically has from 3 to 7, preferably from 3 to 4 carbon atoms. Examples include cyclopropyl, cyclobutyl, cyclopentyl and cyclohexyl. When a cycloalkyl radical carries 2 or more substituents, the substituents may be the same or different. Unless otherwise specified, the substitutents on a cycloalkyl radical are typically themselves unsubstituted.

As used herein, the term heteroaryl radical embraces typically optionally substituted 5-to 10- membered ring systems comprising at least one heteroaromatic ring and containing at least one heteroatom selected from O, S and N. A heteroaryl radical may be a single ring or two or more fused rings wherein at least one ring contains a heteroatom. A said optionally substituted heteroaryl radical is typically unsubstituted or substituted with 1, 2 or 3 substituents which may be the same or different. When a heteroaryl radical carries 2 or more substituents, the substituents may be the same or different. Unless otherwise specified, the substituents on a heteroaryl radical are typically themselves unsubstituted.

Examples include pyridyl, pyrazinyl, pyrimidinyl, pyridazinyl, furyl, benzofuranyl, oxadiazolyl, oxazolyl, isoxazolyl, benzoxazolyl, imidazolyl, benzimidazolyl, thiazolyl, thiadiazolyl, thienyl, pyrrolyl, pyridinyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl, thianthrenyl, pyrazolyl, 2H-pyrazolo[3,4-d]pyrimidinyl, 1H-pyrazolo[3,4-d]pyrimidinyl, thieno[2,3-d] pyrimidnyl and the various pyrrolopyridyl radicals.

As used herein, the term bicyclic N-containing heteroaryl group is typically an optionally substituted, fused 8 to 10 membered ring system comprising at least one heteroatomic ring, containing a nitrogen atom and optionally one or more, for example, 1, 2 or 3, preferably 1, further heteroatoms selected from O, S and N, preferably N. A said optionally substituted bicyclic N-containing heteroaryl group is typically unsubstituted or substituted with 1, 2 or 3 substituents which may be the same or different. When a heteroaryl radical carries 2 or more substituents, the substituents may be the same or different. Unless otherwise specified, the substituents on a heteroaryl radical are typically themselves unsubstituted.

Example include benzofuranyl, benzoxazolyl, benzimidazolyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, indolinyl, isoindolinyl, isoindolyl, pteridinyl, pyrazolopyrimidinyl, thienopyrimidnyl and pyrrolopyridyl. Pyrrolopyridyl is preferred. 1H-pyrrolo-2,3-b]pyridin-1-yl is more preferred.

As used herein, the term heterocyclic radical embraces typically optionally substituted non-aromatic, saturated or unsaturated C₃-C₁₀ carbocyclic ring systems, preferably C₄-C₆ carbocyclic rings, such as 4, 5 or 6 membered radicals, in which one or more, for example 1, 2, or 3 of the carbon atoms preferably 1 or 2 of the carbon atoms are replaced by a heteroatom selected from N, O and S. Saturated heterocyclic radicals are preferred. A heterocyclic radical may be a single ring or two or more fused rings wherein at least one ring contains a heteroatom. When a heterocyclyl radical carries 2 or more substituents, the substituents may be the same or different. Typically, the substituents on a heterocyclyl radical are themselves unsubstituted, unless otherwise specified.

Examples of heterocyclic radicals include azetidyl, piperidyl, pyrrolidyl, pyrrolinyl, piperazinyl, morpholinyl, thiomorpholinyl, pyrazolinyl, pirazolidinyl, and quinuclidinyl.

As used herein, the term saturated N-containing heterocyclic ring is typically a 4 to 6 membered, optionally substituted heterocyclic radical as defined herein, which is a saturated C₄ to C₆ carbocyclic ring, such as a 4, 5 or 6 membered radical, in which one of the carbon atoms is repaced by N and in which, optionally, one or more, for example 1 or 2, preferably 1 further carbon atom is repaced by a heteroatom selected from N, O and S.

Examples include azetidyl, piperidyl, pyrrolidyl, pyrrolinyl, piperazinyl, morpholinyl, thiomorpholinyl, and pirazolidinyl.

As used herein, some of the atoms, radicals, moieties, chains or cycles present in the general structures of the invention are "optionally substituted". This means that these atoms, radicals, moieties, chains or cycles can be either unsubstituted or substituted in any posiition by one or more, for example 1, 2, 3 or 4, substituents, whereby the hydrogen atoms bound to the unsubstituted atoms, radicals, moieties, chains or cycles are replaced by chemically acceptable atoms, radicals, moieties, chains or cycles. When two or more substituents are present, each substituent may be the same or different.

As used herein, the term halogen atom embraces chlorine, fluorine, bromine or iodine atoms typically a fluorine, chlorine or bromine atom, most preferably bromine or fluorine. The term halo when used as a prefix has the same meaning.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X-) is associated with the positive charge on the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

In one embodiment of the present invention, :
A is selected from the group consisting of -N-, -O- and -S-;
B and C are independently selected from the group consisting of -N- and -O-, with the proviso that at least two of A, B and C are nitrogen atoms;
G¹ is selected from the group consisting of -CH₂-, -NH- and -O-;
G² is selected from the group consisting of -NR⁴- and -O-;
R¹ represents:
   ➢ a 8 to 10 membered bicyclic N-containing heteroaryl group optionally substituted with a C₁₋₄ carboxyalkyl group or a C₁₋₄ aminoalkyl group,
   ➢ a pyridyl group optionally substituted with one or more substituents selected from hydroxy groups, C₁₋₄ alkyl groups, C₁₋₄ carboxyalkyl groups, C₁₋₄ haloalkyl groups, C₁₋₄ alkoxy groups, amino groups, C₁₋₄ aminoalkyl groups and C₁₋₄ aminoalkoxy groups,
   ➢ a pyridone group substituted with one or more C₁₋₄ alkyl groups; C₁₋₄ haloalkyl groups or C₁₋₄ aminoalkyl groups, or
   ➢ a group of formula: 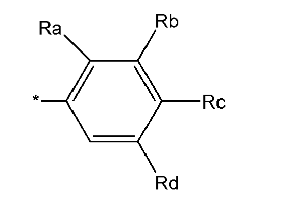 wherein:
      - R^{a} represents a hydrogen atom, a halogen atom, a C₁₋₄ alkyl group, C₃₋₄ cycloalkyl group or a -CF₃ group;
      - R^{b} represents a hydrogen atom, a halogen atom, a C₁₋₄ alkyl group, a -CF₃ group or a C₁₋₄ alkoxy group;
      - R^{d} represents a hydrogen atom, a C₁₋₄ alkyl group or a C₁₋₄ alkoxy group;
      - R^{c} represents:
         o A hydrogen atom, a C₁₋₄ hydroxyalkyl group, a C₁₋₄ aminoalkyl group which is optionally substituted with one or more substituents selected from halogen atoms, hydroxy groups and -CF₃ groups;
         o a 4 to 6-membered saturated N-containing heterocyclic ring optionally substituted with a C₁₋₂ carboxyalkyl group;
         o -(CH₂)₍₀₋₄₎-C(O)OR', -(CH₂)₍₀₋₄₎-C(O)NR'R", -(CH₂)₍₀₋₄₎-NHC(O)R", -S(O)₂NR'R", -O-(CH₂)₍₂₋₄₎NR'R", -O-(CH₂)₍₁₋₄₎C(O)OR", -O-(CH2)₍₁₋₄)-C(O)NR'R", -(CH₂)₍₀₋₄₎-NR'R", -(CH₂)₍₀₋₄₎-CONHS(O)₂R', -(CH₂)₍₀₋₄₎-NHS(O)₂R' or-(CH₂)₍₀₋₃₎-NH-(CH₂)₍₁₋₃₎-(NH)₍₀₋₁₎S(O)₂R' wherein,
            ■ R' represents a hydrogen atom or a C₁₋₄ alkyl group,
            ■ R" represents a hydrogen atom, a C₁₋₄ alkyl group, a C₃₋₄ cycloalkyl group, a C₁₋₄ carboxyalkyl group, a C₁₋₄ haloalkyl group, a C₁₋₄ hydroxyalkyl group or a 6 membered, saturated N-containing heterocyclic ring, or
            ■ R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₄ carboxyalkyl group,
      or R^{c} together with R^{d} form a C₅₋₆ cycloalkyl group optionally substituted by a -NHR^{f} group, wherein R^{f} is selected from the group consisting of a hydrogen atom and a carboxymethyl group;
R² and R³ are independently selected from the group consisting of hydrogen atoms, halogen atoms and C₁₋₄ alkyl groups; and
R^{a} is selected from the group consisting of a hydrogen atom, a phenyl group, a C₃₋₄ cycloalkyl-C₁₋₄ alkyl group, C₁₋₄ aminoalkyl group, C₁₋₄ haloalkyl group and a linear or branched C₁₋₄ alkyl group which is optionally substituted by a phenyl or a pyridyl group.

Typically, in compounds of formula I where R^{a} is a C₃₋₄ cycloalkyl-C₁₋₄ alkyl group, said group is bonded to the nitrogen atom through the alkyl group, i.e. -C₁₋₄ alkyl-C₃₋₄ cycloalkyl.

Typically, when R^{c} represents -(CH₂)₍₀₋₄₎-CONHS(O)₂R', -(CH₂)₍₀₋₄₎-NHS(O)₂R' or - (CH₂)₍₀₋₃₎-NH-(CH₂)₍₁₋₃₎-(NH)₍₀₋₁₎S(O)₂R', then R' is not a hydrogen atom.

Typically, when R^{c} represents -(CH₂)₍₀₋₄₎-NHC(O)R", then R" is not a hydrogen atom.

As used herein, an N-oxide is formed from the tertiary basic amines or imines present in the molecule, using a convenient oxidising agent.

Typically, A is selected from the group consisting of -N- and -O-. Preferably A represents -N-.

More preferably, both A and B represent -N- and C represents -O-.

Typically, G¹ represents a -CH₂- or a -O- group. Preferably G¹ represents a -CH₂-group.

Typically, R² and R³ are independently selected from the group consisting of hydrogen atoms, fluorine atoms and methyl groups. Preferably, both R² and R³ are methyl groups.

Typically, R⁴ is selected from the group consisting of a C₃₋₄ cycloalkyl-C₁₋₄ alkyl group, C₁₋₄ haloalkyl group and a linear or branched unsubstituted C₁₋₄ alkyl group.

Preferably, G² represents -NR⁴-, and R⁴ is selected from the group consisting of a methyl group, ethyl group, t-butyl group, cyclopropylmethyl group and 2,2,2-trifluoroethyl group. More preferably, R⁴ represents a methyl or an ethyl group.

Typically, R¹ represents:
➢ a pyridyl group substituted with one, two or three substituents selected from hydroxy groups and C₁₋₄ alkyl groups;
➢ a pyridone group substituted with one or two C₁₋₂ alkyl groups; or
➢ a group of formula: 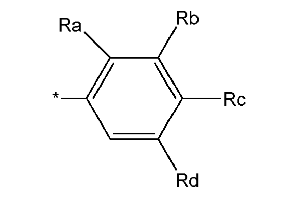 wherein:
   - R^{a} represents a hydrogen atom or a C₁₋₄ alkyl group;
   - R^{b} represents a hydrogen atom or C₁₋₄ alkyl group;
   - R^{d} represents a hydrogen atom or a C₁₋₄ alkyl group;
   - R^{c} represents:
      o a C₁₋₄ hydroxyalkyl group or a C₁₋₄ aminoalkyl substitued by one or more halogen atoms;
      o -(CH₂)₍₂₋₃₎-C(O)OR', -(CH₂)₍₀₋₂₎-C(O)NR'R", -O-(CH₂)₍₂₋₃₎NR'R", -(CH₂)₍₂₋₃₎-NHC(O)R", -S(O)₂NR'R", -(CH₂)₍₀₋₃₎-NR'R" or -(CH₂)₍₁₋₂₎-CONHS(O)₂R' wherein,
         ■ R' represents a hydrogen atom or a methyl group,
         ■ R" represents a hydrogen atom, a C₁₋₄ alkyl group, C₁₋₄ carboxyalkyl group, C₁₋₄ haloalkyl group or a C₁₋₄ hydroxyalkyl group, or
         ■ R' and R" together with the nitrogen atom to which they are attached from a 4 to 6 membered heterocyclic group which contains, as heteroatoms, one N atom and, optionally, one further N atom, and which is optionally substituted with a carboxy or a C₁₋₂ carboxyalkyl group,
      or R^{c} together with R^{d} form a cyclohexyl group substituted with a carboxymethylamino group.

Preferably, R¹ represents:
➢ a pyridyl group substituted with two or three substituents selected from hydroxy groups, methyl and ethyl groups, or
➢ a group of formula: 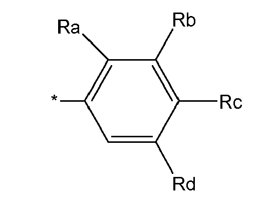 wherein:
   - R^{a} represents a hydrogen atom or a methyl group;
   - R^{b} represents a hydrogen atom, a methyl group;
   - R^{d} represents a hydrogen atom or a methyl group;
   - R^{c} represents: -(CH₂)₍₂₋₃₎-C(O)OR', -(CH₂)₂-C(O)NR'R" or -(CH₂)₍₂₋₃₎-NR'R", wherein
      o R' represents a hydrogen atom;
      o R" represents a hydrogen atom, a C₁₋₂ carboxyalkyl group, a C₁₋₄ haloalkyl group or a C₁₋₂ hydroxyalkyl group; or
      o R' and R" together with the nitrogen atom to which they are attached form a 4 membered saturated heterocyclic group, which contains as heteroatom, one nitrogen atom and which is substituted with a carboxy group.

More preferably, R¹ represents a group of formula: 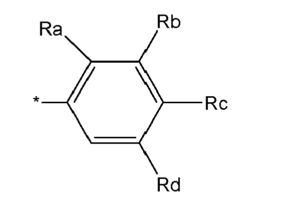 wherein:
o R^{a} represents a hydrogen atom;
o both R^{b} and R^{d} represent methyl groups; and
o R^{c} represents -(CH₂)₍₂₋₃₎-C(O)OH or -(CH₂)₍₂₋₃₎-NHR", wherein R" is selected from a hydrogen atom, a C₁₋₂ carboxyalkyl group, and C₁₋₂ hydroxyalkyl group.

Typically, G¹ represents a -CH₂- group, G² represents a -NR₄- group, wherein R⁴ represents a methyl or ethyl group, both R² and R³ represent a methyl group, and
R¹ represents:
   ➢ a pyridyl group substituted with two or three substituents selected from hydroxy groups, methyl or ethyl groups, or
   ➢ a group of formula: 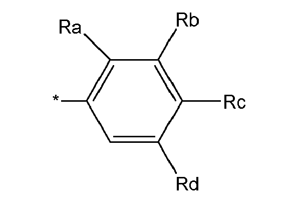 wherein:
      - R^{a} represents a hydrogen atom or a methyl group;
      - R^{b} represents a hydrogen atom, a methyl group,
      - R^{d} represents a hydrogen atom or a methyl group,
      - R^{c} represents: -(CH₂)₍₂₋₃₎-C(O)OR', -(CH₂)₂-C(O)NR'R" or -(CH₂)₍₂₋₃₎-NR'R", wherein:
         o R' represents a hydrogen atom;
         o R" represents a hydrogen atom, a C₁₋₂ carboxyalkyl group, a C₁₋₄ haloalkyl group or a C₁₋₂ hydroxyalkyl group, or
         o R' and R" together with the nitrogen atom to which they are attached form a 4 membered saturated heterocyclic group, which contains as heteroatom, one nitrogen atom and which is substituted with a carboxy group.

More preferably, R¹ represents a group of formula: 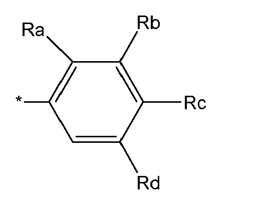 wherein
o R^{a} represents a hydrogen atom;
o both R^{b} and R^{d} represents a methyl group and
o R^{c} represents -(CH₂)₍₂₋₃₎-C(O)OH or -(CH₂)₍₂₋₃₎-NHR", wherein R" is selected from a hydrogen atom, a C₁₋₂ carboxyalkyl group and C₁₋₂ hydroxyalkyl group.

Typically, R¹ represents:
➢ an imidazo[1,2-a]pyridyl group or a 3H-pyrrolo[2,3-b]pyridyl group which are optionally substituted with a carboxyethyl group;
➢ a pyridyl group optionally substituted with one or more substituents selected from hydroxy groups, methyl groups, ethyl groups, carboxyethyl groups, -CF₃ groups, methoxy groups and amino groups
➢ a pyridone group substituted with one or more substituents selected from methyl and ethyl groups; or
➢ a group of formula: 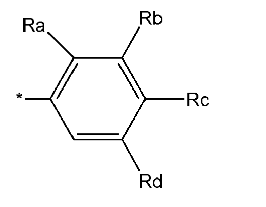 wherein:
   - R^{a} represents a hydrogen atom, a methyl group, cyclopropyl group or a CF₃ group;
   - R^{b} represents a hydrogen atom, a chlorine atom or a methyl group;
   - R^{d} represents a hydrogen atom or a methyl group;
   - R^{c} represents:
      o a C₁₋₄ hydroxyalkyl group or a C₁₋₄ aminoalkyl group substituted with one or more substituents selected from fluorine atoms and hydroxy groups;
      o a 4 to 6-membered saturated N-containing heterocyclic ring which is optionally substituted with a C₁₋₂ carboxyalkyl group
      o -(CH₂)₍₀₋₄₎-C(O)OR', -(CH₂)₍₀₋₄₎-C(O)NR'R", -(CH₂)₍₀₋₄₎-NHC(O)R", -S(O)₂NR'R", -O-(CH₂)₍₂₋₄₎NR'R", -O-(CH₂)₍₁₋₄₎C(O)OR", -O-(CH₂)₍₁₋₄₎-C(O)NR'R" -(CH₂)₍₀₋₄₎-NR'R", -(CH₂)₍₀₋₄₎-CONHS(O)₂R' -(CH₂)₍₀₋₄₎-NHS(O)₂R' or -(CH₂)₍₀₋₃₎-NH-(CH₂)₍₁₋₃₎-(NH)₍₀₋₁₎S(O)₂R' wherein
      o R' represents a hydrogen atom or a methyl group,
      o R" represents a hydrogen atom, a methyl group, a cyclopropyl group, a piperidyl group, a C₁₋₂ carboxyalkyl group, a CF₃ group, C₁₋₄ hydroxyalkyl group, or
      o R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₄ carboxyalkyl group,
or R^{c} together with R^{d} form a cyclohexyl group optionally substituted by a -NHR^{f} group, wherein R^{f} is selected from the group consisting of a hydrogen atom and a carboxymethyl group;
R² and R³ are independently selected from the group consisting of hydrogen atoms, fluorine atoms and methyl groups; and
R⁴ is selected from the group consisting of a hydrogen atom, a phenyl group, a C₃₋₄ cycloalkyl-C₁₋₂ alkyl group, C₁₋₂ aminoalkyl group, C₁₋₂ haloalkyl group or R⁴ represents a linear or branched C₁₋₄ alkyl group which is optionally substituted by a phenyl group or a pyridyl group.

Typically, R¹ represents a group of formula: 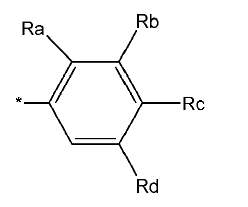 wherein:
- R^{a} represents a hydrogen atom,
- R^{b} represents a methyl group or a CF₃ group,
- R^{d} represents a hydrogen atom or a methyl group;
- R^{c} represents a -(CH₂)₍₀₋₄₎-C(O)NR'R", -(CH₂)₍₀₋₄₎-NHC(O)R" or -(CH₂)₍₀₋₄₎-NR'R", wherein
   o R' represents a hydrogen atom or a methyl group,
   o R" represents a hydrogen atom, a methyl group, a C₁₋₂ carboxyalkyl group or a C₁₋₄ hydroxyalkyl group, or
   o R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₄ carboxyalkyl group

More preferably, R^{c} represents a -(CH₂)₍₂₋₃₎-NR'R", wherein R' and R" together with the nitrogen atom to which they are attached from a 4 to 6 membered heterocyclic group which contains, as heteroatoms, one N atom, and which is optionally substituted with a carboxy or a C₁₋₂ carboxyalkyl group.

Preferred compounds of the S1 P1 receptor agonist of the invention are represented by the formula (I'), or a pharmaceutically acceptable salt, or solvate, or N-oxide, or stereoisomer or deuterated derivate thereof: 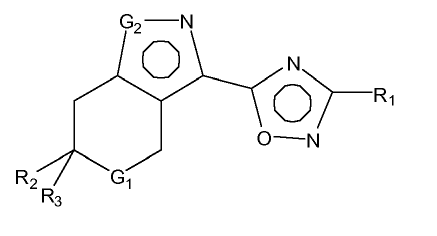 Formula (I')
wherein,
G¹ is selected from the group consisting of -CH₂-, and -O-;
G² is selected from the group consisting of -NR⁴- and -O-;
R¹ represents:
➢ A pyrrolopyridyl group, which is unsubstituted or substituted with a C₁₋₂ carboxyalkyl group;
➢ a pyridyl group optionally substituted with 1, 2 or 3 substituents selected from hydroxy groups, C₁₋₂ alkyl groups, C₁₋₂ carboxyalkyl groups, C₁₋₂ haloalkyl groups, C₁₋₂ alkoxy groups, and amino groups;
➢ a pyridone group substituted with 1, 2 or 3 C₁₋₂ alkyl groups; or
➢ a group of formula: 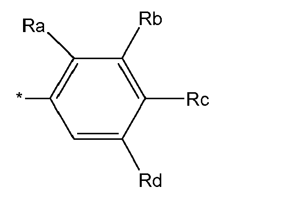 wherein:
   - R^{a} represents a hydrogen atom, a C₁₋₂ alkyl group, a cyclopropyl group or a -CF₃ group;
   - R^{b} represents a hydrogen atom, a chlorine atom, or a C₁₋₂ alkyl group;
   - R^{d} represents a hydrogen atom, or a C₁₋₂ alkyl group;
   - R^{c} represents:
      o a C₁₋₃ hydroxyalkyl group;
      o a carboxyethylpiperazine group;
      o -(CH₂)₍₀₋₂₎-C(O)OR', -(CH₂)₍₀₋₂₎-C(O)NR'R", -S(O)₂NR'R", or -(CH₂)₍₀₋₄₎-NR'R", wherein,
         ■ R' represents a hydrogen atom,
         ■ R" represents a hydrogen atom, a C₁₋₂ alkyl group, a cyclopropyl group, a C₁₋₂ carboxyalkyl group, a C₁₋₂ haloalkyl group, a C₁₋₂ hydroxyalkyl group or a piperidyl group, or
         ■ R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₂ carboxyalkyl group,
      or R^{c} together with R^{d} forms a cyclohexyl group substituted by a -NHR^{f} group, wherein R^{f} is a carboxymethyl group;
R² and R³ are independently selected from the group consisting of hydrogen atoms, fluorine atoms and C₁₋₂ alkyl groups; and
R⁴ is selected from the group consisting of hydrogen atoms, phenyl groups, cyclopropyl-C₁₋₂ alkyl groups, C₁₋₂ aminoalkyl groups, C₁₋₂ haloalkyl groups and linear or branched C₁₋₄ alkyl groups which are optionally substituted by a phenyl or a pyridyl group,
wherein said pyrrolopyridyl groups are typically 1H-pyrrolo-[2, 3-b]pyridyl groups.

Preferably, the S1 P1 receptor agonist of the present invention is one of the following list:
1. 4-(5-(6,6-Dimethyl-4,5,6,7-tetrahydro-2H-indazol-3-yl)-1,2,4-oxadiazol-3-yl)benzenesulfonamide,
2. (4-(5-(6,6-Dimethyl-4,5,6,7-tetrahydro-2H-indazol-3-yl)-1,2,4-oxadiazol-3-yl)phenyl)methanol,
3. (4-(5-(1,6,6-Trimethyl-4,5,6,7-tetrahydro-1H-indazol-3-yl)-1,2,4-oxadiazol-3-yl)phenyl)methanol,
4. 4-(5-(1,6,6-Trimethyl-4,5,6,7-tetrahydro-1H-indazol-3-yl)-1,2,4-oxadiazol-3-yl)benzenesulfonamide,
5. 4-(5-(1-Ethyl-6,6-dimethyl-4,5,6,7-tetrahydro-1H-indazol-3-yl)-1,2,4-oxadiazol-3-yl)benzenesulfonamide
6. 5-(1-Ethyl-6,6-dimethyl-4,5,6,7-tetrahydro-1H-indazol-3-yl)-3-(pyridin-4-yl)-1,2,4-oxadiazole,
7. 3-(4-(5-(1-Ethyl-6,6-dimethyl-4,5,6,7-tetrahydro-1H-indazol-3-yl)-1,2,4-oxadiazol-3-yl)-2,6-dimethylphenyl)propanoic acid,
8. 3-(4-(4-(5-(1-Ethyl-6,6-dimethyl-4,5,6,7-tetrahydro-1H-indazol-3-yl)-1,2,4-oxadiazol-3-yl)phenyl)piperazin-1-yl)propanoic acid,
9. 4-(5-(1-Ethyl-6,6-dimethyl-4,5,6,7-tetrahydro-1H-indazol-3-yl)-1,2,4-oxadiazol-3-yl)benzoic acid,
10. 4-(5-(1-Ethyl-6,6-dimethyl-4,5,6,7-tetrahydro-1H-indazol-3-yl)-1,2,4-oxadiazol-3-yl)benzamide,
11. 5-(1-Ethyl-6,6-dimethyl-4,5,6,7-tetrahydro-1H-indazol-3-yl)-3-(4-(piperazin-1-yl)phenyl)-1,2,4-oxadiazole,
12. 2-(4-(5-(1-Ethyl-6,6-dimethyl-4,5,6,7-tetrahydro-1H-indazol-3-yl)-1,2,4-oxadiazol-3-yl)phenyl)acetic acid,
13. 1-(4-(5-(1-Ethyl-6,6-dimethyl-4,5,6,7-tetrahydro-1H-indazol-3-yl)-1,2,4-oxadiazol-3-yl)benzyl)azetidine-3-carboxylic acid,
14. 2-(4-(5-(1-Ethyl-6,6-dimethyl-4,5,6,7-tetrahydro-1H-indazol-3-yl)-1,2,4-oxadiazol-3-yl)phenyl)acetamide,
15. 4-(5-(1-Ethyl-6,6-dimethyl-4,5,6,7-tetrahydro-1H-indazol-3-yl)-1,2,4-oxadiazol-3-yl)pyridine 1-oxide,
16. N-(4-(5-(1-Ethyl-6,6-dimethyl-4,5,6,7-tetrahydro-1H-indazol-3-yl)-1,2,4-oxadiazol-3-yl)phenyl)piperidin-4-amine,
17. 5-(1-Ethyl-6,6-dimethyl-4,5,6,7-tetrahydro-1H-indazol-3-yl)-3-(6-methoxypyridin-3-yl)-1,2,4-oxadiazole,
18. 5-(5-(1-Ethyl-6,6-dimethyl-4,5,6,7-tetrahydro-1H-indazol-3-yl)-1,2,4-oxadiazol-3-yl)pyridin-2-ol,
19. 4-(5-(1-Ethyl-6,6-dimethyl-4,5,6,7-tetrahydro-1H-indazol-3-yl)-1,2,4-oxadiazol-3-yl)pyridin-2-ol,
20. 3-(4-(5-(1-Ethyl-6,6-dimethyl-4,5,6,7-tetrahydro-1H-indazol-3-yl)-1,2,4-oxadiazol-3-yl)-2,6-dimethylphenyl)propanamide,
21. 4-(5-(1-Benzyl-6,6-dimethyl-4,5,6,7-tetrahydro-1H-indazol-3-yl)-1,2,4-oxadiazol-3-yl)benzamide,
22. 4-(5-(1-tert-Butyl-6,6-dimethyl-4,5,6,7-tetrahydro-1H-indazol-3-yl)-1,2,4-oxadiazol-3-yl)benzamide,
23. 5-(1-Ethyl-6,6-dimethyl-4,5,6,7-tetrahydro-1H-indazol-3-yl)-3-(2-methoxypyridin-4-yl)-1,2,4-oxadiazole,
24. 4-(5-(1-Ethyl-6,6-dimethyl-4,5,6,7-tetrahydro-1H-indazol-3-yl)-1,2,4-oxadiazol-3-yl)-1-methylpyridin-2(1H)-one,
25. 1-(4-(5-(1-Ethyl-6,6-dimethyl-4,5,6,7-tetrahydro-1H-indazol-3-yl)-1,2,4-oxadiazol-3-yl)phenyl)piperidine-4-carboxylic acid,
26. (4-(5-(1-Ethyl-6,6-dimethyl-4,5,6,7-tetrahydro-1H-indazol-3-yl)-1,2,4-oxadiazol-3-yl)phenyl)methanol,
27. 4-(5-(6,6-Dimethyl-1-phenyl-4,5,6,7-tetrahydro-1H-indazol-3-yl)-1,2,4-oxadiazol-3-yl)benzamide,
28. 3-Ethyl-5-(5-(1-ethyl-6,6-dimethyl-4,5,6,7-tetrahydro-1H-indazol-3-yl)-1,2,4-oxadiazol-3-yl)-6-methylpyridin-2-ol,
29. 5-(5-(1-Ethyl-6,6-dimethyl-4,5,6,7-tetrahydro-1H-indazol-3-yl)-1,2,4-oxadiazol-3-yl)-3-methylpyridin-2-ol,
30. 4-(5-(1-Ethyl-6,6-dimethyl-4,5,6,7-tetrahydro-1H-indazol-3-yl)-1,2,4-oxadiazol-3-yl)-3-methylbenzamide,
31. 4-(4-(5-(1-Ethyl-6,6-dimethyl-4,5,6,7-tetrahydro-1H-indazol-3-yl)-1,2,4-oxadiazol-3-yl)benzyl)morpholine,
32. 3-Ethyl-5-(5-(1-ethyl-6,6-dimethyl-4,5,6,7-tetrahydro-1H-indazol-3-yl)-1,2,4-oxadiazol-3-yl)-1,6-dimethylpyridin-2(1H)-one,
33. 5-(5-(1-Ethyl-6,6-dimethyl-4,5,6,7-tetrahydro-1H-indazol-3-yl)-1,2,4-oxadiazol-3-yl)-1,3-dimethylpyridin-2(1H)-one,
34. N-Cyclopropyl-4-(5-(1-ethyl-6,6-dimethyl-4,5,6,7-tetrahydro-1H-indazol-3-yl)-1,2,4-oxadiazol-3-yl)benzamide,
35. 1-(4-(5-(1-Ethyl-6,6-dimethyl-4,5,6,7-tetrahydro-1H-indazol-3-yl)-1,2,4-oxadiazol-3-yl)phenyl)-N-methylmethanamine,
36. 5-(1-Ethyl-6,6-dimethyl-4,5,6,7-tetrahydro-1H-indazol-3-yl)-3-(3-methylpyridin-4-yl)-1,2,4-oxadiazole,
37. 4-(5-(6,6-Dimethyl-1-(2,2,2-trifluoroethyl)-4,5,6,7-tetrahydro-1H-indazol-3-yl)-1,2,4-oxadiazol-3-yl)benzamide,
38. (4-(5-(1-Ethyl-6,6-dimethyl-4,5,6,7-tetrahydro-1H-indazol-3-yl)-1,2,4-oxadiazol-3-yl)phenyl)methanamine,
39. 5-(1-Ethyl-6,6-dimethyl-4,5,6,7-tetrahydro-1H-indazol-3-yl)-3-(3-methylpyridin-2-yl)-1,2,4-oxadiazole,
40. 2-(4-(5-(1-Ethyl-6,6-dimethyl-4,5,6,7-tetrahydro-1H-indazol-3-yl)-1,2,4-oxadiazol-3-yl)-2,6-dimethylphenyl)ethanamine,
41. 5-(5-(1-Ethyl-6,6-dimethyl-4,5,6,7-tetrahydro-1H-indazol-3-yl)-1,2,4-oxadiazol-3-yl)-4-methylpyridin-2-ol,
42. 3-(4-(5-(1-Ethyl-6,6-dimethyl-4,5,6,7-tetrahydro-1H-indazol-3-yl)-1,2,4-oxadiazol-3-yl)-3-methylphenyl)propanoic acid,
43. 5-(5-(1-Ethyl-6,6-dimethyl-4,5,6,7-tetrahydro-1H-indazol-3-yl)-1,2,4-oxadiazol-3-yl)-6-methylpyridin-2-ol,
44. 5-(1-Ethyl-6,6-dimethyl-4,5,6,7-tetrahydro-1H-indazol-3-yl)-3-(4-methylpyridin-3-yl)-1,2,4-oxadiazole,
45. 5-(1-Ethyl-6,6-dimethyl-4,5,6,7-tetrahydro-1H-indazol-3-yl)-3-(4-(trifluoromethyl)pyridin-3-yl)-1,2,4-oxadiazole,
46. 5-(1-Ethyl-6,6-dimethyl-4,5,6,7-tetrahydro-1H-indazol-3-yl)-3-(imidazo[1,2-a]pyridin-6-yl)-1,2,4-oxadiazole,
47. 3-(4-(5-(1-Ethyl-6,6-dimethyl-4,5,6,7-tetrahydro-1H-indazol-3-yl)-1,2,4-oxadiazol-3-yl)-3-methylphenyl)propanamide,
48. 2-(4-(5-(1-Ethyl-6,6-dimethyl-4,5,6,7-tetrahydro-1H-indazol-3-yl)-1,2,4-oxadiazol-3-yl)-3-methylphenyl)ethanamine,
49. 3-(4-(5-(1-Ethyl-6,6-dimethyl-4,5,6,7-tetrahydro-1H-indazol-3-yl)-1,2,4-oxadiazol-3-yl)-2,6-dimethylphenyl)propan-1-amine,
50. 4-(5-(1-Ethyl-6,6-dimethyl-4,5,6,7-tetrahydro-1H-indazol-3-yl)-1,2,4-oxadiazol-3-yl)-3-(trifluoromethyl)benzoic acid,
51. 4-(5-(1-Ethyl-6,6-dimethyl-4,5,6,7-tetrahydro-1H-indazol-3-yl)-1,2,4-oxadiazol-3-yl)-3-(trifluoromethyl)benzamide,
52. 5-(1-Ethyl-6,6-dimethyl-4,5,6,7-tetrahydro-1H-indazol-3-yl)-3-(2-methylpyridin-3-yl)-1,2,4-oxadiazole,
53. 5-(5-(1-Ethyl-6,6-dimethyl-4,5,6,7-tetrahydro-1H-indazol-3-yl)-1,2,4-oxadiazol-3-yl)-6-(trifluoromethyl)pyridin-2-amine,
54. 3-Cyclopropyl-4-[5-(1-ethyl-6,6-dimethyl-4,5,6,7-tetrahydro-1H-indazol-3-yl)-1,2,4-oxadiazol-3-yl]benzamide,
55. 5-[5-(1-Ethyl-6,6-dimethyl-4,5,6,7-tetrahydro-1H-indazol-3-yl)-1,2,4-oxadiazol-3-yl]-6-(trifluoromethyl)pyridin-2-ol,
56. 5-(5-(6,6-Dimethyl-1-(2,2,2-trifluoroethyl)-4,5,6,7-tetrahydro-1H-indazol-3-yl)-1,2,4-oxadiazol-3-yl)-3-ethyl-6-methylpyridin-2-ol,
57. 3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propanoic acid,
58. 3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propanamide,
59. 3-Ethyl-5-[5-(1-ethyl-6,6-dimethyl-1,4,6,7-tetrahydropyrano[4,3-c]pyrazol-3-yl)-1,2,4-oxadiazol-3-yl]-6-methylpyridin-2-ol,
60. 3-(4-(5-(1-Ethyl-6,6-dimethyl-4,5,6,7-tetrahydro-1H-indazol-3-yl)-1,2,4-oxadiazol-3-yl)-3-methylphenyl)propan-1-amine,
61. 3-(4-(5-(1-Ethyl-6,6-dimethyl-4,5,6,7-tetrahydro-1 H-indazol-3-yl)-1,2,4-oxadiazol-3-yl)-3-(trifluoromethyl)phenyl)propan-1-amine,
62. 6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-amine,
63. 2-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)ethanamine,
64. 3-(2,6-Dimethyl-4-(5-(1,6,6-trimethyl-4,5,6,7-tetrahydro-1H-indazol-3-yl)-1,2,4-oxadiazol-3-yl)phenyl)propanoic acid,
65. 3-(2,6-Dimethyl-4-(5-(1,6,6-trimethyl-4,5,6,7-tetrahydro-1H-indazol-3-yl)-1,2,4-oxadiazol-3-yl)phenyl)propanamide,
66. 3-(4-(5-(6,6-Dimethyl-4,5,6,7-tetrahydrobenzo[d]isoxazol-3-yl)-1,2,4-oxadiazol-3-yl)-2,6-dimethylphenyl)propanoic acid,
67. 3-(4-(5-(6,6-Dimethyl-4,5,6,7-tetrahydrobenzo[d]isoxazol-3-yl)-1,2,4-oxadiazol-3-yl)-2,6-dimethylphenyl)propanamide,
68. 2-(6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-ylamino)ethanoic acid,
69. 3-(2,6-Dimethyl-4-(5-(1,6,6-trimethyl-4,5,6,7-tetrahydro-1H-indazol-3-yl)-1,2,4-oxadiazol-3-yl)phenyl)propan-1-amine,
70. 3-(4-(5-(6,6-Dimethyl-4,5,6,7-tetrahydrobenzo[d]isoxazol-3-yl)-1,2,4-oxadiazol-3-yl)-2,6-dimethylphenyl)propan-1-amine,
71. 2-(2,6-Dimethyl-4-(5-(1,6,6-trimethyl-4,5,6,7-tetrahydro-1H-indazol-3-yl)-1,2,4-oxadiazol-3-yl)phenyl)ethanamine,
72. 2-(4-(5-(6,6-Dimethyl-4,5,6,7-tetrahydrobenzo[d]isoxazol-3-yl)-1,2,4-oxadiazol-3-yl)-2,6-dimethylphenyl)ethanamine,
73. 5-(5-(6,6-Dimethyl-1-(pyridin-3-ylmethyl)-4,5,6,7-tetrahydro-1H-indazol-3-yl)-1,2,4-oxadiazol-3-yl)-3-ethyl-6-methylpyridin-2(1H)-one,
74. 2-(Dimethyl-4,5,6,7-tetrahydrobenzo[d]isoxazol-3-yl)-1,2,4-oxadiazol-3-yl)-2,6-dimethylphenethylamino)ethanoic acid,
75. 2-(3-(4-(5-(6,6-Dimethyl-4,5,6,7-tetrahydrobenzo[d]isoxazol-3-yl)-1,2,4-oxadiazol-3-yl)-2,6-dimethylphenyl)propylamino)ethanoic acid,
76. 3-(Dimethyl-4,5,6,7-tetrahydrobenzo[d]isoxazol-3-yl)-1,2,4-oxadiazol-3-yl)-2,6-dimethylphenethylamino)propanoic acid,
77. 3-Ethyl-5-(5-(1-ethyl-4,5,6,7-tetrahydro-1H-indazol-3-yl)-1,2,4-oxadiazol-3-yl)-6-methylpyridin-2(1H)-one,
78. 3-Ethyl-6-methyl-5-(5-(1,6,6-trimethyl-4,5,6,7-tetrahydro-1H-indazol-3-yl)-1,2,4-oxadiazol-3-yl)pyridin-2-ol,
79. 5-(5-(6,6-Dimethyl-4,5,6,7-tetrahydrobenzo[d]isoxazol-3-yl)-1,2,4-oxadiazol-3-yl)-3-ethyl-6-methylpyridin-2(1H)-one,
80. 5-(5-(1-(2-Aminoethyl)-6,6-dimethyl-4,5,6,7-tetrahydro-1H-indazol-3-yl)-1,2,4-oxadiazol-3-yl)-3-ethyl-6-methylpyridin-2-ol,
81. 3-Ethyl-5-(5-(1-ethyl-6,6-dimethyl-4,5,6,7-tetrahydro-1H-indazol-3-yl)-1,2,4-oxadiazol-3-yl)-6-methylpyridin-2-amine,
82. 2-(4-(5-(6,6-Dimethyl-4,5,6,7-tetrahydrobenzo[d]isoxazol-3-yl)-1,2,4-oxadiazol-3-yl)-2,6-dimethylphenyl)-N,N-dimethylethanamine,
83. 3-(4-(4-(5-(6,6-Dimethyl-4,5,6,7-tetrahydrobenzo[d]isoxazol-3-yl)-1,2,4-oxadiazol-3-yl)phenyl)piperazin-1-yl)propanoic acid,
84. 3-Ethyl-5-(5-(1-ethyl-6,6-difluoro-4,5,6,7-tetrahydro-1H-indazol-3-yl)-1,2,4-oxadiazol-3-yl)-6-methylpyridin-2(1H)-one,
85. 3-(3-Ethyl-5-(5-(1-ethyl-6,6-dimethyl-4,5,6,7-tetrahydro-1H-indazol-3-yl)-1,2,4-oxadiazol-3-yl)-6-methylpyridin-2-yl)propanoic acid,
86. 5-(5-(1-(Cyclopropylmethyl)-6,6-dimethyl-4,5,6,7-tetrahydro-1H-indazol-3-yl)-1,2,4-oxadiazol-3-yl)-3-ethyl-6-methylpyridin-2(1H)-one,
87. 3-(2,6-Dimethyl-4-(5-(1,6,6-trimethyl-4,5,6,7-tetrahydro-1H-indazol-3-yl)-1,2,4-oxadiazol-3-yl)phenyl)propane-1,2-diol,
88. N-(2,6-Dimethyl-4-(5-(1,6,6-trimethyl-4,5,6,7-tetrahydro-1H-indazol-3-yl)-1,2,4-oxadiazol-3-yl)phenethyl)-2,2,2-trifluoroethanamine,
89. 2-(2,6-Dimethyl-4-(5-(1,6,6-trimethyl-4,5,6,7-tetrahydro-1H-indazol-3-yl)-1,2,4-oxadiazol-3-yl)phenethylamino)ethanol,
90. 2-(2,6-Dimethyl-4-(5-(1,6,6-trimethyl-4,5,6,7-tetrahydro-1H-indazol-3-yl)-1,2,4-oxadiazol-3-yl)phenethylamino)ethanoic acid,
91. 1-(2,6-Dimethyl-4-(5-(1,6,6-trimethyl-4,5,6,7-tetrahydro-1H-indazol-3-yl)-1,2,4-oxadiazol-3-yl)phenethyl)azetidine-3-carboxylic acid,
92. 3-(2-Methyl-4-(5-(1,6,6-trimethyl-4,5,6,7-tetrahydro-1H-indazol-3-yl)-1,2,4-oxadiazol-3-yl)phenyl)propanoic acid,
93. 4-(2,6-Dimethyl-4-(5-(1,6,6-trimethyl-4,5,6,7-tetrahydro-1H-indazol-3-yl)-1,2,4-oxadiazol-3-yl)phenethyl)morpholine,
94. 3-(4-(5-(6,6-Dimethyl-4,5,6,7-tetrahydro-1H-indazol-3-yl)-1,2,4-oxadiazol-3-yl)-2,6-dimethylphenyl)propane-1,2-diol,
95. 3-(4-(5-(1-Ethyl-6,6-dimethyl-4,5,6,7-tetrahydro-1H-indazol-3-yl)-1,2,4-oxadiazol-3-yl)phenylamido)propanoic acid,
96. 3-(4-(5-(6,6-Dimethyl-1-(pyridin-3-ylmethyl)-4,5,6,7-tetrahydro-1H-indazol-3-yl)-1,2,4-oxadiazol-3-yl)-2,6-dimethylphenyl)propane-1,2-diol,
97. 3-(2-Chloro-4-(5-(1-ethyl-6,6-dimethyl-4,5,6,7-tetrahydro-1H-indazol-3-yl)-1,2,4-oxadiazol-3-yl)phenylsulfonamido)propanoic acid,
98. 3-(4-(5-(6,6-Dimethyl-4,5,6,7-tetrahydrobenzo[d]isoxazol-3-yl)-1,2,4-oxadiazol-3-yl)-2,6-dimethylphenyl)propane-1,2-diol,
99. 3-(1-Ethyl-6,6-dimethyl-4,5,6,7-tetrahydro-1H-indazol-3-yl)-5-(pyridin-4-yl)-1,2,4-oxadiazole,
100. 5-(1-Ethyl-6,6-dimethyl-4,5,6,7-tetrahydro-1H-indazol-3-yl)-3-o-tolyl-1,2,4-oxadiazole,
101. 3-(5-(5-(1-Ethyl-6,6-dimethyl-4,5,6,7-tetrahydro-1H-indazol-3-yl)-1,2,4-oxadiazol-3-yl)-1H-pyrrolo[2,3-b]pyridin-1-yl)propanoic acid,
102. 3-(5-(5-(6,6-Dimethyl-4,5,6,7-tetrahydrobenzo[d]isoxazol-3-yl)-1,2,4-oxadiazol-3-yl)-1H-pyrrolo[2,3-b]pyridin-1-yl)propanoic acid,
103. 1-amino-3-{2,6-dimethyl-4-[5-(1,6,6-trimethyl-4,5,6,7-tetrahydro-1H-indazol-3-yl)-1,2,4-oxadiazol-3-yl]phenyl}propan-2-ol,
104. N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-2-methylpropan-2-amine,
105. 3-{4-[3-(1-ethyl-6,6-dimethyl-4,5,6,7-tetrahydro-1H-indazol-3-yl)-1,2,4-oxadiazol-5-yl]-3-methylphenyl}propanoic acid,
106. [2-(4-{5-[6,6-dimethyl-1-(pyridin-3-ylmethyl)-4,5,6,7-tetrahydro-1H-indazol-3-yl]-1,2,4-oxadiazol-3-yl}-2,6-dimethylphenyl)ethyl]amine,
107. 3-(4-{5-[1-(cyclopropylmethyl)-6,6-dimethyl-4,5,6,7-tetrahydro-1H-indazol-3-yl]-1,2,4-oxadiazol-3-yl}-2,6-dimethylphenyl)propanoic acid,
108. (2-{4-[3-(1-ethyl-6,6-dimethyl-4,5,6,7-tetrahydro-1H-indazol-3-yl)-1,2,4-oxadiazol-5-yl]-3-methylphenyl}ethyl)amine,
109. N-[2-(4-{5-[6,6-dimethyl-1-(pyridin-3-ylmethyl)-4,5,6,7-tetrahydro-1H-indazol-3-yl]-1,2,4-oxadiazol-3-yl}-2,6-dimethylphenyl)ethyl]glycine,
110. 3-{4-[5-(1-ethyl-6,6-difluoro-4,5,6,7-tetrahydro-1H-indazol-3-yl)-1,2,4-oxadiazol-3-yl]-2,6-dimethylphenyl}propanoic acid,
111. 3-(4-{5-[6,6-dimethyl-1-(pyridin-3-ylmethyl)-4,5,6,7-tetrahydro-1H-indazol-3-yl]-1,2,4-oxadiazol-3-yl}-2,6-dimethylphenyl)propanoic acid,
112. N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-2-hydroxyacetamide,
113. 3-{4-[5-(1-ethyl-6,6-dimethyl-4,5,6,7-tetrahydro-1H-indazol-3-yl)-1,2,4-oxadiazol-3-yl]-2,6-dimethylphenyl}-N-(methylsulfonyl)propanamide,
114. 3-{4-[5-(1-ethyl-6,6-dimethyl-4,5,6,7-tetrahydro-1H-indazol-3-yl)-1,2,4-oxadiazol-3-yl]-2-methylphenyl}propanoic acid,
115. 3-{4-[5-(1-ethyl-6,6-dimethyl-4,5,6,7-tetrahydro-1H-indazol-3-yl)-1,2,4-oxadiazol-3-yl]-2,6-dimethylphenyl}propane-1,2-diol,
116. N-(2-{4-[5-(6,6-dimethyl-4,5,6,7-tetrahydro-1H-indazol-3-yl)-1,2,4-oxadiazol-3-yl]-2,6-dimethylphenyl}ethyl)-2,2,2-trifluoroethanamine,
117. N-({4-[5-(1-ethyl-6,6-dimethyl-4,5,6,7-tetrahydro-1H-indazol-3-yl)-1,2,4-oxadiazol-3-yl]-3-methylphenyl}sulfonyl)-beta-alanine,
118. N-(3-{4-[5-(1-ethyl-6,6-dimethyl-4,5,6,7-tetrahydro-1H-indazol-3-yl)-1,2,4-oxadiazol-3-yl]-2,6-dimethylphenyl}propyl)methanesulfonamide,
119. N-(3-{2,6-dimethyl-4-[5-(1,6,6-trimethyl-4,5,6,7-tetrahydro-1H-indazol-3-yl)-1,2,4-oxadiazol-3-yl]phenyl}propanoyl)glycine,
120. (2-{4-[5-(1-ethyl-6,6-dimethyl-4,5,6,7-tetrahydro-1H-indazol-3-yl)-1,2,4-oxadiazol-3-yl]-2-methylphenyl}ethyl)amine,
121. N-({4-[5-(1-ethyl-6,6-dimethyl-4,5,6,7-tetrahydro-1H-indazol-3-yl)-1,2,4-oxadiazol-3-yl]-3-methylphenyl}sulfonyl)glycine,
122. 1-ethyl-6,6-dimethyl-3-[3-(2-methyl-4-piperidin-4-ylphenyl)-1,2,4-oxadiazol-5-yl]-4,5,6,7-tetrahydro-1H-indazole,
123. (4-{4-[5-(1-ethyl-6,6-dimethyl-4,5,6,7-tetrahydro-1H-indazol-3-yl)-1,2,4-oxadiazol-3-yl]-3-methylphenyl}piperidin-1-yl)acetic acid,
124. 3-(4-{4-[5-(1-ethyl-6,6-dimethyl-4,5,6,7-tetrahydro-1H-indazol-3-yl)-1,2,4-oxadiazol-3-yl]-3-methylphenyl}piperidin-1-yl)propanoic acid,
125. (2-{4-[3-(1-ethyl-6,6-dimethyl-4,5,6,7-tetrahydro-1H-indazol-3-yl)-1,2,4-oxadiazol-5-yl]-2,6-dimethylphenoxy}ethyl)amine,
126. 3-{4-[5-(1-ethyl-6,6-dimethyl-4,5,6,7-tetrahydro-1H-indazol-3-yl)-1,2,4-oxadiazol-3-yl]-2,5-dimethylphenyl}propanoic acid,
127. {4-[3-(1-ethyl-6,6-dimethyl-4,5,6,7-tetrahydro-1H-indazol-3-yl)-1,2,4-oxadiazol-5-yl]-2,6-dimethylphenoxy}acetic acid,
128. 3-{4-[3-(1-ethyl-6,6-dimethyl-4,5,6,7-tetrahydro-1H-indazol-3-yl)-1,2,4-oxadiazol-5-yl]-2,6-dimethoxyphenyl}propanoic acid,
129. 3-{2-chloro-4-[3-(1-ethyl-6,6-dimethyl-4,5,6,7-tetrahydro-1H-indazol-3-yl)-1,2,4-oxadiazol-5-yl]-6-methoxyphenyl}propanoic acid,
130. (2-{2,6-dimethyl-4-[5-(1,6,6-trimethyl-4,5,6,7-tetrahydro-1H-indazol-3-yl)-1,2,4-oxadiazol-3-yl]phenyl}ethyl)[2-(methylsulfonyl)ethyl]amine,
131. 3-{2-ethyl-4-[5-(1-ethyl-6,6-dimethyl-4,5,6,7-tetrahydro-1H-indazol-3-yl)-1,2,4-oxadiazol-3-yl]-6-methylphenyl}propanoic acid,
132. (2-{3-(trifluoromethyl)-4-[5-(1,6,6-trimethyl-4,5,6,7-tetrahydro-1H-indazol-3-yl)-1,2,4-oxadiazol-3-yl]phenyl}ethyl)amine,
133. {4-[5-(1-ethyl-6,6-dimethyl-4,5,6,7-tetrahydro-1H-indazol-3-yl)-1,2,4-oxadiazol-3-yl]-2,6-dimethylphenyl}acetic acid,
134. [3-({3-ethyl-5-[5-(1-ethyl-6,6-dimethyl-4,5,6,7-tetrahydro-1H-indazol-3-yl)-1,2,4-oxadiazol-3-yl]-6-methylpyridin-2-yl}oxy)propyl]amine,
135. 1,6,6-trimethyl-3-[3-(2-methyl-4-piperidin-4-ylphenyl)-1,2,4-oxadiazol-5-yl]-4,5,6,7-tetrahydro-1 H-indazole,
136. 2-{4-[5-(1-ethyl-6,6-dimethyl-4,5,6,7-tetrahydro-1H-indazol-3-yl)-1,2,4-oxadiazol-3-yl]-2,6-dimethylphenyl}acetamide,
137. 2-{4-[5-(1-ethyl-6,6-dimethyl-4,5,6,7-tetrahydro-1H-indazol-3-yl)-1,2,4-oxadiazol-3-yl]-2,6-dimethylphenyl}acetamide,
138. (2-{3-ethyl-5-[5-(1-ethyl-6,6-dimethyl-4,5,6,7-tetrahydro-1H-indazol-3-yl)-1,2,4-oxadiazol-3-yl]-6-methylpyridin-2-yl}ethyl)amine,
139. (2-{2,6-dimethyl-4-[5-(1,6,6-trimethyl-4,5,6,7-tetrahydro-1H-indazol-3-yl)-1,3,4-thiadiazol-2-yl]phenoxy}ethyl)amine,
140. {2-[4-[5-(1-ethyl-6,6-dimethyl-4,5,6,7-tetrahydro-1H-indazol-3-yl)-1,2,4-oxadiazol-3-yl]-2-(trifluoromethyl)phenyl]ethyl}amine,
141. 2-{2,6-dimethyl-4-[5-(1,6,6-trimethyl-4,5,6,7-tetrahydro-1H-indazol-3-yl)-1,2,4-oxadiazol-3-yl]phenyl}ethanol,
142. 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid,
143. [1-(2-{2,6-dimethyl-4-[5-(1,6,6-trimethyl-4,5,6,7-tetrahydro-1H-indazol-3-yl)-1,2,4-oxadazol-3-yl]phenyl}ethyl)piperidin-4-yl]acetic acid,
144. 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)pyrrolidine-3-carboxylic acid,
145. (3S)-1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)pyrrolidine-3-carboxylic acid,
146. N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-L-alanine,
147. N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-2-methylalanine,
148. N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-D-alanine,
149. 2-((2,6-dimethyl-4-(5-(1,6,6-trimethyl-4,5,6,7-tetrahydro-1H-indazol-3-yl)-1,2,4-oxadiazol-3-yl)phenethyl)(methyl)amino)acetic acid,
150. (2-{2,6-dimethyl-4-[5-(1,6,6-trimethyl-4,5,6,7-tetrahydro-1H-indazol-3-yl)-1,3,4-oxadiazol-2-yl]phenoxy}ethyl)amine,
151. {2-[4-[5-(1-ethyl-6,6-dimethyl-4,5,6,7-tetrahydro-1H-indazol-3-yl)-1,2,4-oxadiazol-3-yl]-2-(trifluoromethyl)phenoxy]ethyl}amine,
152. (2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)amine,
153. (2-{2-(trifluoromethyl)-4-[5-(1,6,6-trimethyl-4,5,6,7-tetrahydro-1H-indazol-3-yl)-1,2,4-oxadiazol-3-yl]phenoxy}ethyl)amine,
154. (2,2-difluoro-2-{2-methyl-4-[5-(1,6,6-trimethyl-4,5,6,7-tetrahydro-1H-indazol-3-yl)-1,2,4-oxadiazol-3-yl]phenyl}ethyl)amine,
155. 1-{2,6-dimethyl-4-[5-(1,6,6-trimethyl-4,5,6,7-tetrahydro-1H-indazol-3-yl)-1,2,4-oxadiazol-3-yl]phenyl}propan-2-ol,
156. 3-{2,6-dimethyl-4-[5-(1,6,6-trimethyl-4,5,6,7-tetrahydro-1H-indazol-3-yl)-1,2,4-oxadiazol-3-yl]phenyl}propan-1-ol,,
157. [4-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperazin-1-yl]acetic acid,
158. 1-(2-{4-[5-(1-ethyl-6,6-dimethyl-4,5,6,7-tetrahydro-1H-indazol-3-yl)-1,2,4-oxadiazol-3-yl]-2,6-dimethylphenyl}ethyl)piperidine-4-carboxylic acid,
159. 1-(2-{4-[5-(1-ethyl-6,6-dimethyl-4,5,6,7-tetrahydro-1H-indazol-3-yl)-1,2,4-oxadiazol-3-yl]-2-methylphenyl}ethyl)piperidine-4-carboxylic acid,
160. 1-(2-{4-[3-(1-ethyl-6,6-dimethyl-4,5,6,7-tetrahydro-1H-indazol-3-yl)-1,2,4-oxadiazol-5-yl]-2-methylphenyl}ethyl)piperidine-4-carboxylic acid,
161. 1-{2-[4-[5-(1-ethyl-6,6-dimethyl-4,5,6,7-tetrahydro-1H-indazol-3-yl)-1,2,4-oxadiazol-3-yl]-2-(trifluoromethyl)phenyl]ethyl}piperidine-4-carboxylic acid,
162. N-{2-[4-[5-(1-ethyl-6,6-dimethyl-4,5,6,7-tetrahydro-1H-indazol-3-yl)-1,2,4-oxadiazol-3-yl]-2-(trifluoromethyl)phenyl]ethyl}glycine,
163. 4-[5-(1,6,6-trimethyl-4,5,6,7-tetrahydro-1H-indazol-3-yl)-1,2,4-oxadiazol-3-yl]benzoic acid,
164. 1-(2-{3-methyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid,
165. (1-{2-[4-[5-(1-ethyl-6,6-dimethyl-4,5,6,7-tetrahydro-1H-indazol-3-yl)-1,2,4-oxadiazol-3-yl]-2-(trifluoromethyl)phenyl]ethyl}piperidin-4-yl)acetic acid,
166. 2-[(2-{4-[5-(6,6-dimethyl-4,5,6,7-tetrahydro-1,2-benzisoxazol-3-yl)-1,2,4-oxadiazol-3-yl]-2,6-dimethylphenyl}ethyl)amino]ethanol,
167. N-{2-[(2-{4-[5-(6,6-dimethyl-4,5,6,7-tetrahydro-1,2-benzisoxazol-3-yl)-1,2,4-oxadiazol-3-yl]-2,6-dimethylphenyl}ethyl)amino]ethyl}methane-sulfonamide,
168. N-(2-{4-[5-(6,6-dimethyl-4,5,6,7-tetrahydro-1,2-benzisoxazol-3-yl)-1,2,4-oxadiazol-3-yl]-2,6-dimethylphenyl}ethyl)-2,2,2-trifluoroethanamine,
169. 1-(2-{4-[5-(6,6-dimethyl-4,5,6,7-tetrahydro-1,2-benzisoxazol-3-yl)-1,2,4-oxadiazol-3-yl]-2,6-dimethylphenyl}ethyl)piperidine-4-carboxylic acid,
170. 3-{4-[5-(6,6-dimethyl-4,5,6,7-tetrahydro-1,2-benzisoxazol-3-yl)-1,2,4-oxadiazol-3-yl]-2,6-dimethylphenyl}propane-1,2-diol,
171. 1-(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)piperidine-4-carboxylic acid,
172. 2-(2-(trifluoromethyl)-4-(5-(1,6,6-trimethyl-4,5,6,7-tetrahydro-1H-indazol-3-yl)-1,2,4-oxadiazol-3-yl)phenethylamino)acetic acid,
173. 2-(methyl(2-(trifluoromethyl)-4-(5-(1,6,6-trimethyl-4,5,6,7-tetrahydro-1H-indazol-3-yl)-1,2,4-oxadiazol-3-yl)phenethyl)amino)acetic acid,
174. 1-(2-(trifluoromethyl)-4-(5-(1,6,6-trimethyl-4,5,6,7-tetrahydro-1H-indazol-3-yl)-1,2,4-oxadiazol-3-yl)phenethyl)azetidine-3-carboxylic acid,
175. 2-(1-(2-(trifluoromethyl)-4-(5-(1,6,6-trimethyl-4,5,6,7-tetrahydro-1H-indazol-3-yl)-1,2,4-oxadiazol-3-yl)phenethyl)piperidin-4-yl)acetic acid,
176. 1-(2,6-dimethyl-4-(5-(1,6,6-trimethyl-4,5,6,7-tetrahydro-1H-indazol-3-yl)-4H-1,2,4-triazol-3-yl)phenethyl)piperidine-4-carboxylic acid,
177. 1-(2,6-dimethyl-4-(5-(1,6,6-trimethyl-4,5,6,7-tetrahydro-1H-indazol-3-yl)-1,3,4-oxadiazol-2-yl)phenethyl)piperidine-4-carboxylic acid,
178. 1-(2,6-dimethyl-4-(5-(1,6,6-trimethyl-4,5,6,7-tetrahydro-1H-indazol-3-yl)-1,3,4-thiadiazol-2-yl)phenethyl)piperidine-4-carboxylic acid,
179. 2-(1-(2,6-dimethyl-4-(5-(1,6,6-trimethyl-4,5,6,7-tetrahydro-1H-indazol-3-yl)-1,3,4-thiadiazol-2-yl)phenethyl)piperidin-4-yl)acetic acid,
180. 1-(2-(trifluoromethyl)-4-(5-(1,6,6-trimethyl-4,5,6,7-tetrahydro-1H-indazol-3-yl)-1,3,4-oxadiazol-2-yl)phenethyl)piperidine-4-carboxylic acid,
181. 1-(2-(trifluoromethyl)-4-(5-(1,6,6-trimethyl-4,5,6,7-tetrahydro-1H-indazol-3-yl)-1,3,4-thiadiazol-2-yl)phenethyl)piperidine-4-carboxylic acid,
182. 2-(1-(2-(trifluoromethyl)-4-(5-(1,6,6-trimethyl-4,5,6,7-tetrahydro-1H-indazol-3-yl)-1,3,4-thiadiazol-2-yl)phenethyl)piperidin-4-yl)acetic acid,
183. 2-(5-(5-(1-ethyl-6,6-dimethyl-4,5,6,7-tetrahydro-1H-indazol-3-yl)-1,2,4-oxadiazol-3-yl)-1H-pyrrolo[2,3-b]pyridin-1-yl)ethanamine,
184. 5-(1-ethyl-6,6-dimethyl-4,5,6,7-tetrahydro-1H-indazol-3-yl)-3-(1H-pyrrolo[2,3-b]pyridin-5-yl)-1,2,4-oxadiazole,
185. 2-(5-(5-(1,6,6-trimethyl-4,5,6,7-tetrahydro-1H-indazol-3-yl)-1,2,4-oxadiazol-3-yl)-1H-pyrrolo[2,3-b]pyridin-1-yl)ethanamine,
186. 3-(1H-indazol-5-yl)-5-(1,6,6-trimethyl-4,5,6,7-tetrahydro-1H-indazol-3-yl)-1,2,4-oxadiazole,
187. 5-(1-ethyl-6,6-dimethyl-4,5,6,7-tetrahydro-1H-indazol-3-yl)-3-(1H-indol-4-yl)-1,2,4-oxadiazole,
188. 3-(1H-indol-4-yl)-5-(1,6,6-trimethyl-4,5,6,7-tetrahydro-1H-indazol-3-yl)-1,2,4-oxadiazole,
189. 5-(1-ethyl-6,6-dimethyl-4,5,6,7-tetrahydro-1H-indazol-3-yl)-3-(1H-indol-5-yl)-1,2,4-oxadiazole,
190. 3-(1H-benzo[d]imidazol-5-yl)-5-(1-ethyl-6,6-dimethyl-4,5,6,7-tetrahydro-1H-indazol-3-yl)-1,2,4-oxadiazole,
191. 2-(4-(5-(1-ethyl-6,6-dimethyl-4,5,6,7-tetrahydro-1H-indazol-3-yl)-1,2,4-oxadiazol-3-yl)-1H-indol-1-yl)acetic acid,
192. 2-(5-(5-(1,6,6-trimethyl-4,5,6,7-tetrahydro-1H-indazol-3-yl)-1,2,4-oxadiazol-3-yl)-1 H-indol-1-yl)acetic acid,
193. 3-(4-(5-(1-ethyl-6,6-dimethyl-4,5,6,7-tetrahydro-1H-indazol-3-yl)-1,2,4-oxadiazol-3-yl)-1 H-indol-1-yl)propanoic acid,
194. 2-(5-(5-(1-ethyl-6,6-dimethyl-4,5,6,7-tetrahydro-1H-indazol-3-yl)-1,2,4-oxadiazol-3-yl)-1H-indol-1-yl)acetic acid,
195. 3-(5-(5-(1-ethyl-6,6-dimethyl-4,5,6,7-tetrahydro-1H-indazol-3-yl)-1,2,4-oxadiazol-3-yl)-1H-indol-1-yl)propanoic acid,
196. 2-(1-(2-(trifluoromethyl)-4-(5-(1,6,6-trimethyl-4,5,6,7-tetrahydro-1H-indazol-3-yl)-1,3,4-oxadiazol-2-yl)phenethyl)piperidin-4-yl)acetic acid,
197. 2-(2-(trifluoromethyl)-4-(5-(1,6,6-trimethyl-4,5,6,7-tetrahydro-1H-indazol-3-yl)-1,3,4-thiadiazol-2-yl)phenethylamino)acetic acid,
198. 1-(2-(trifluoromethyl)-4-(5-(1,6,6-trimethyl-4,5,6,7-tetrahydro-1H-indazol-3-yl)-1,3,4-thiadiazol-2-yl)phenethyl)azetidine-3-carboxylic acid,
199. 1-(2-(trifluoromethyl)-4-(3-(1,6,6-trimethyl-4,5,6,7-tetrahydro-1H-indazol-3-yl)-1,2,4-oxadiazol-5-yl)phenethyl)piperidine-4-carboxylic acid
200. 2-(1-(2-(trifluoromethyl)-4-(3-(1,6,6-trimethyl-4,5,6,7-tetrahydro-1H-indazol-3-yl)-1,2,4-oxadiazol-5-yl)phenethyl)piperidin-4-yl)acetic acid
201. 3-(3-(trifluoromethyl)-4-(5-(1,6,6-trimethyl-4,5,6,7-tetrahydro-1H-indazol-3-yl)-1,2,4-oxadiazol-3-yl)phenyl)propanoic acid,
202. 3-(4-(5-(1-ethyl-6,6-dimethyl-4,5,6,7-tetrahydro-1H-indazol-3-yl)-1,2,4-oxadiazol-3-yl)-2-(trifluoromethyl)phenyl)propanoic acid, and
203. 3-(2-(trifluoromethyl)-4-(5-(1,6,6-trimethyl-4,5,6,7-tetrahydro-1H-indazol-3-yl)-1,2,4-oxadiazol-3-yl)phenyl)propanoic acid
or a pharmaceutically acceptable salt, or solvate, or N-oxide, or stereoisomer or deuterated derivate thereof,

More preferably, the S1P1 receptor agonist is one of:
4-(5-(1-Ethyl-6,6-dimethyl-4,5,6,7-tetrahydro-1H-indazol-3-yl)-1,2,4-oxadiazol-3-yl)benzenesulfonamide,
3-(4-(5-(1-Ethyl-6,6-dimethyl-4,5,6,7-tetrahydro-1H-indazol-3-yl)-1,2,4-oxadiazol-3-yl)-2,6-dimethylphenyl)propanoic acid,
3-(4-(4-(5-(1-Ethyl-6,6-dimethyl-4,5,6,7-tetrahydro-1H-indazol-3-yl)-1,2,4-oxadiazol-3-yl)phenyl)piperazin-1-yl)propanoic acid,
4-(5-(1-Ethyl-6,6-dimethyl-4,5,6,7-tetrahydro-1H-indazol-3-yl)-1,2,4-oxadiazol-3-yl)benzamide,
1-(4-(5-(1-Ethyl-6,6-dimethyl-4,5,6,7-tetrahydro-1H-indazol-3-yl)-1,2,4-oxadiazol-3-yl)benzyl)azetidine-3-carboxylic acid,
2-(4-(5-(1-Ethyl-6,6-dimethyl-4,5,6,7-tetrahydro-1H-indazol-3-yl)-1,2,4-oxadiazol-3-yl)phenyl)acetamide,
3-(4-(5-(1-Ethyl-6,6-dimethyl-4,5,6,7-tetrahydro-1H-indazol-3-yl)-1,2,4-oxadiazol-3-yl)-2,6-dimethylphenyl)propanamide,
4-(5-(1-Benzyl-6,6-dimethyl-4,5,6,7-tetrahydro-1H-indazol-3-yl)-1,2,4-oxadiazol-3-yl)benzamide,
4-(5-(1-tert-Butyl-6,6-dimethyl-4,5,6,7-tetrahydro-1H-indazol-3-yl)-1,2,4-oxadiazol-3-yl)benzamide,
3-Ethyl-5-(5-(1-ethyl-6,6-dimethyl-4,5,6,7-tetrahydro-1H-indazol-3-yl)-1,2,4-oxadiazol-3-yl)-6-methylpyridin-2-ol,
5-(5-(1-Ethyl-6,6-dimethyl-4,5,6,7-tetrahydro-1H-indazol-3-yl)-1,2,4-oxadiazol-3-yl)-3-methylpyridin-2-ol,
4-(5-(1-Ethyl-6,6-dimethyl-4,5,6,7-tetrahydro-1H-indazol-3-yl)-1,2,4-oxadiazol-3-yl)-3-methylbenzamide
5-(1-Ethyl-6,6-dimethyl-4,5,6,7-tetrahydro-1H-indazol-3-yl)-3-(3-methylpyridin-4-yl)-1,2,4-oxadiazole,
4-(5-(6,6-Dimethyl-1-(2,2,2-trifluoroethyl)-4,5,6,7-tetrahydro-1H-indazol-3-yl)-1,2,4-oxad iazol-3-yl)benzam ide,
2-(4-(5-(1-Ethyl-6,6-dimethyl-4,5,6,7-tetrahydro-1H-indazol-3-yl)-1,2,4-oxadiazol-3-yl)-2,6-dimethylphenyl)ethanamine,
3-(4-(5-(1-Ethyl-6,6-dimethyl-4,5,6,7-tetrahydro-1H-indazol-3-yl)-1,2,4-oxadiazol-3-yl)-3-methylphenyl)propanoic acid,
5-(5-(1-Ethyl-6,6-dimethyl-4,5,6,7-tetrahydro-1H-indazol-3-yl)-1,2,4-oxadiazol-3-yl)-6-methylpyridin-2-ol,
3-(4-(5-(1-Ethyl-6,6-dimethyl-4,5,6,7-tetrahydro-1H-indazol-3-yl)-1,2,4-oxadiazol-3-yl)-3-methylphenyl)propanamide,
2-(4-(5-(1-Ethyl-6,6-dimethyl-4,5,6,7-tetrahydro-1H-indazol-3-yl)-1,2,4-oxadiazol-3-yl)-3-methylphenyl)ethanamine,
3-(4-(5-(1-Ethyl-6,6-dimethyl-4,5,6,7-tetrahydro-1H-indazol-3-yl)-1,2,4-oxadiazol-3-yl)-2,6-dimethylphenyl)propan-1-amine,
5-(5-(6,6-Dimethyl-1-(2,2,2-trifluoroethyl)-4,5,6,7-tetrahydro-1H-indazol-3-yl)-1,2,4-oxadiazol-3-yl)-3-ethyl-6-methylpyridin-2-ol,
6-(5-(-Ethyl-6,6-dimethyl-4,5,6,7-tetrahydro-1H-indazol-3-yl)-1,2,4-oxadiazol-3-yl)-1,2,3,4-tetrahydronaphthalen-2-amine,
3-(2,6-Dimethyl-4-(5-(1,6,6-trimethyl-4,5,6,7-tetrahydro-1H-indazol-3-yl)-1,2,4-oxadiazol-3-yl)phenyl)propanamide,
3-(4-(5-(6,6-Dimethyl-4,5,6,7-tetrahydrobenzo[d]isoxazol-3-yl)-1,2,4-oxadiazol-3-yl)-2,6-dimethylphenyl)propanamide,
2-(6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-ylamino)ethanoic acid,
3-(2,6-Dimethyl-4-(5-(1,6,6-trimethyl-4,5,6,7-tetrahydro-1H-indazol-3-yl)-1,2,4-oxadiazol-3-yl)phenyl)propan-1-amine,
3-(4-(5-(6,6-Dimethyl-4,5,6,7-tetrahydrobenzo[d]isoxazol-3-yl)-1,2,4-oxadiazol-3-yl)-2,6-dimethylphenyl)propan-1-amine,
2-(2,6-Dimethyl-4-(5-(1,6,6-trimethyl-4,5,6,7-tetrahydro-1H-indazol-3-yl)-1,2,4-oxadiazol-3-yl)phenyl)ethanamine,
2-(4-(5-(6,6-Dimethyl-4,5,6,7-tetrahydrobenzo[d]isoxazol-3-yl)-1,2,4-oxadiazol-3-yl)-2,6-dimethylphenyl)ethanamine,
2-(Dimethyl-4,5,6,7-tetrahydrobenzo[d]isoxazol-3-yl)-1,2,4-oxadiazol-3-yl)-2,6-dimethylphenethylamino)ethanoic acid,
2-(3-(4-(5-(6,6-Dimethyl-4,5,6,7-tetrahydrobenzo[d]isoxazol-3-yl)-1,2,4-oxadiazol-3-yl)-2,6-dimethylphenyl)propylamino)ethanoic acid,
3-(Dimethyl-4,5,6,7-tetrahydrobenzo[d]isoxazol-3-yl)-1,2,4-oxadiazol-3-yl)-2,6-dimethylphenethylamino)propanoic acid,
5-(5-(1-(Cyclopropylmethyl)-6,6-dimethyl-4,5,6,7-tetrahydro-1H-indazol-3-yl)-1,2,4-oxadiazol-3-yl)-3-ethyl-6-methylpyridin-2(1H)-one,
3-(2,6-Dimethyl-4-(5-(1,6,6-trimethyl-4,5,6,7-tetrahydro-1H-indazol-3-yl)-1,2,4-oxadiazol-3-yl)phenyl)propane-1,2-diol,
N-(2,6-Dimethyl-4-(5-(1,6,6-trimethyl-4,5,6,7-tetrahydro-1H-indazol-3-yl)-1,2,4-oxadiazol-3-yl)phenethyl)-2,2,2-trifluoroethanamine,
2-(2,6-Dimethyl-4-(5-(1,6,6-trimethyl-4,5,6,7-tetrahydro-1H-indazol-3-yl)-1,2,4-oxadiazol-3-yl)phenethylamino)ethanol,
2-(2,6-Dimethyl-4-(5-(1,6,6-trimethyl-4,5,6,7-tetrahydro-1H-indazol-3-yl)-1,2,4-oxadiazol-3-yl)phenethylamino)ethanoic acid,
1-(2,6-Dimethyl-4-(5-(1,6,6-trimethyl-4,5,6,7-tetrahydro-1H-indazol-3-yl)-1,2,4-oxadiazol-3-yl)phenethyl)azetidine-3-carboxylic acid,
1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid,
N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-2-methylpropan-2-amine,
3-{4-[3-(1-ethyl-6,6-dimethyl-4,5,6,7-tetrahydro-1H-indazol-3-yl)-1,2,4-oxadiazol-5-yl]-3-methylphenyl}propanoic acid,
[2-(4-{5-[6,6-dimethyl-1-(pyridin-3-ylmethyl)-4,5,6,7-tetrahydro-1H-indazol-3-yl]-1,2,4-oxadiazol-3-yl}-2,6-dimethylphenyl)ethyl]amine,
N-({4-[5-(1-ethyl-6,6-dimethyl-4,5,6,7-tetrahydro-1H-indazol-3-yl)-1,2,4-oxadiazol-3-yl]-3-methylphenyl}sulfonyl)-beta-alanine,
2-{4-[5-(1-ethyl-6,6-dimethyl-4,5,6,7-tetrahydro-1H-indazol-3-yl)-1,2,4-oxadiazol-3-yl]-2,6-dimethylphenyl}acetamide,
(2-{3-ethyl-5-[5-(1-ethyl-6,6-dimethyl-4,5,6,7-tetrahydro-1H-indazol-3-yl)-1,2,4-oxadiazol-3-yl]-6-methylpyridin-2-yl}ethyl)amine,
(2-{2,6-dimethyl-4-[5-(1,6,6-thmethyl-4,5,6,7-tetrahydro-1H-indazol-3-yl)-1,3,4-thiadiazol-2-yl]phenoxy}ethyl)amine,
2-{2,6-dimethyl-4-[5-(1,6,6-trimethyl-4,5,6,7-tetrahydro-1H-indazol-3-yl)-1,2,4-oxadiazol-3-yl]phenyl}ethanol,
[1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidin-4-yl]acetic acid
N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-L-alanine,
3-{4-[5-(6,6-dimethyl-4,5,6,7-tetrahydro-1,2-benzisoxazol-3-yl)-1,2,4-oxadiazol-3-yl]-2,6-dimethylphenyl}propane-1,2-diol
5-(1-ethyl-6,6-dimethyl-4,5,6,7-tetrahydro-1H-indazol-3-yl)-3-(1H-indol-4-yl)-1,2,4-oxadiazole,
1-(2-(trifluoromethyl)-4-(5-(1,6,6-trimethyl-4,5,6,7-tetrahydro-1H-indazol-3-yl)-1,3,4-thiadiazol-2-yl)phenethyl)azetidine-3-carboxylic acid and
1-(2-{2-(trifluoromethyl)-4-[5-(1,6,6-trimethyl-4,5,6,7-tetrahydro-1H-indazol-3-yl)-1,2,4-oxadiazol-3-yl)phenyl}ethyl)piperidine-4-carboxylicacid,
or a pharmaceutically acceptable salt, or solvate, or N-oxide, or stereoisomer or deuterated derivate thereof.

Most preferably, the S1P1 receptor agonist is 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid or 1-(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)piperidine-4-carboxylic acid, or a pharmaceutically acceptable salt, or solvate, or N-oxide, or stereoisomer or deuterated thereof. More preferably the S1P1 receptor agonists are in the form of hydrochloride salt.

In a particularly preferred embodiment, the S1P1 receptor agonist is 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid or a pharmaceutically acceptable salt, or solvate, or N-oxide, or stereoisomer or deuterated thereof. More preferably the S1P1 receptor agonist is in the form of hydrochloride salt.

In another perferred embodiment, the S1P1 receptor agonist is 1-(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)piperidine-4-carboxylic acid, or a pharmaceutically acceptable salt, or solvate, or N-oxide, or stereoisomer or deuterated thereof. More preferably the S1P1 receptor agonist is in the form of hydrochloride salt.

The present invention further provides the use of a S1P1 receptor agonist of the invention for the manufacture of a medicament for the treatment of Crohn's disease. Typically, the S1P1 receptor agonist is selected from 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid or 1-(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)piperidine-4-carboxylic acid, or or a pharmaceutically acceptable salt, or solvate, or N-oxide, or stereoisomer or deuterated derivate thereof. More preferably the S1P1 receptor agonists are in the form of hydrochloride salt.

In a particularly preferred embodiment, the S1P1 receptor agonist used in the manufacture of a medicament for the treatment of Crohn's disease is 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid or a pharmaceutically acceptable salt, or solvate, or N-oxide, or stereoisomer or deuterated thereof. More preferably the S1P1 receptor agonist is in the form of hydrochloride salt.

In another perferred embodiment, the S1P1 receptor used in the manufacture of a medicament for the treatment of Crohn's disease is 1-(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)piperidine-4-carboxylic acid, or a pharmaceutically acceptable salt, or solvate, or N-oxide, or stereoisomer or deuterated derivate thereof. More preferably the S1P1 receptor agonist is in the form of hydrochloride salt.

fThe present invention also provides a combination of a S1P1 receptor agonist of formula (I) together with another active compound for use in the treatment of Crohn's disease, wherein the other active compound is selected from:
(a) Anti-TNF alpha agents such as infliximab and adalimimab,
(b) immunosuppressants such as azathioprine and 6-mercapthopurine,
(c) anti-inflammatory agents such as sulphasalazine,
(d) anti-alpha 4 integrin antibodies, such as Natalizumab (Tysabri),
(e) alpha 4 integrin antagonists such as R-1295 , TBC-4746, CDP-323, ELND-002, Firategrast and TMC-2003,
(f) ,dyhydrofolate reductase inhibitors, such as Methotrexate or CH-1504,
(g) glucocorticoids such as prednisone or methylprednisolone,
(h) DHODH inhibitors such as Teriflunomide and compounds disclosed in WO 2008/077639 and WO2009021696.
(i) cannabinoid receptor agonists such as Sativex,
(j) chemokine CCR1 antagonists such as MLN-3897 or PS-031291.

Also provided is a product comprising (a) a S1P1 receptor agonist of the invention and (b) another compound as defined above, as a combined preparation for simultaneous, separate or sequential use in the treatment of Crohn's disease.

Also provided is a kit of parts comprising (a) a S1P1 receptor agonist of the invention together with instructions for simultaneous, separate or sequential use in combination with (b) another compound as defined above, for the treatment of Crohn's disease..

Also provided is a package comprising (a) a S1P1 receptor agonist of the invention and (b) another compound as defined above, for simultaneous, separate or sequential use in the treatment of Crohn's disease.

Typically the medicament is for use in treating a human or animal patient suffering or susceptible to hepatic impairment or a condition that would be aggravated by hepatotoxicity. More typically, the said human or animal patient is suffering from liver fibrosis, hepatitis (typically hepatitis A to G), cirrhosis (typically caused by alcoholism) or liver cancer.

The fact that the S1P1 agonist of the invention have reduced hepatic side effects is a finding of the invention. The present invention therefore also provides the use of a S1P1 agonist of formula (I), in the manufacture of a medicament for use in treating or preventing a pathological condition or disease, as defined above, in a human or animal patient which is suffering from or susceptible to hepatic impairment or a condition that would be aggravated by hepatotoxicity.

Also provided is a method of treating a human or animal patient suffering from or susceptible to a pathological condition or disease as defined above, which method comprises administering to said human or animal patient a therapeutically effective amount of a S1P1 receptor agonist as defined above.

Also provided is a method of treating Crohn's disease wherein the human or animal patient is suffering from or susceptible to hepatic impairment or a condition that would be aggravated by hepatotoxicity as defined above, which method comprises administering to said human or animal patient a therapeutically effective amount of a S1P1 receptor agonist as defined above.

The S1P1 receptor agonist of the invention may be administered by any suitable route, depending on the nature of the disorder to be treated, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The active compound in the combination, i.e. the sphingosine-1-phosphate agonist of the invention, and the other optional active compounds as defined above may be administered together in the same pharmaceutical composition or in different compositions intended for separate, simultaneous, concomitant or sequential administration by the same or a different route.

The pharmaceutical formulations may conveniently be presented in unit dosage form and may be prepared by any of the methods well known in the art of pharmacy.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 2µg and 150 µg of each therapeutically active ingredient. Alternatively, the active ingredient (s) may be presented without excipients.

Packaging of the formulation for inhalation may be carried out by using suitable inhaler devices such as the Genuair® (formerly known as Novolizer SD2FL) which is described in the following patent applications: WO 97/000703, WO 03/000325 and WO 06/008027.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

The amount of each active which is required to achieve a therapeutic effect will, of course, vary with the particular active, the route of administration, the subject under treatment, and the particular disorder or disease being treated.

In one embodiment of the present invention, the effective doses of the S1P1 agonist of the present invention is administered via oral route.

Effective doses are normally in the range of 0.5-2000 mg per day of the active ingredient of the present invention, i.e. the S1P1 agonist receptor of formula (I). Daily dosage may be administered in one or more treatments, preferably from 1 to 4 treatments, per day. Preferably, the active ingredients are administered once or twice a day.

### PHARMACOLOGICAL ACTIVITY

### Example 1: 35S-GTP-g binding assay:

The effect of the compounds was measured using a 35S-GTPyS binding assay. Briefly, membranes were incubated in a buffer containing 20 mM HEPES pH 7.4, 100 mM NaCl, 10 mM MgCl2, 10µM GDP, 50µg/ml saponin and 0.2% fatty acid-free BSA at various concentrations (0.1nM-10µM) and 0.1nM 35S-GTPyS. 10µM S1P1 was used as 100% maximum efficacy. The assay was incubated for 90 min at room temperature with gentle mixing, and terminated by filtrating the reaction mixture through GF/C filter plates using the Manifold Filtration System. The filters were immediately washed with sodium phosphate pH 7.4 buffers. After drying the filter plates scintillant liquid were added to each well and 35S-GTPyS binding was measured on a Trilux Scintillation Counter.

The results are shown in Table 1.

**Table 1**

| **Compounds** | **EC₅₀ (nM)** |
|---|---|
| 5 | 40 |
| 8 | 17 |
| 21 | 18 |
| 36 | 48 |
| 42 | 16 |
| 48 | 8 |
| 56 | 31 |
| 68 | 1.9 |
| 74 | 1.7 |
| 91 | 16 |
| 97 | 26 |
| 99 | 146 |
| 104 | 8.5 |
| 117 | 11.7 |
| 137 | 3.6 |
| 138 | 13 |
| 139 | 7.3 |
| 141 | 4.6 |
| 142 | 8.7 |
| 143 | 5.1 |
| 146 | 1.4 |
| 162 | 0.56 |
| 170 | 8.3 |
| 171 | 18.8 |
| 187 | 10 |
| 198 | 12 |

### Example 2 - Hepatotoxicity assesment

Acute hepatotoxicity assays were performed in Swiss mice. Animals received a single administration of either vehicle, or Fingolimod or a compound of the present invention (compounds from the list indicated previously) by intraperitoneal route. Twenty-four hours later, animals were sacrificed and the levels of liver markers ALT (alanine aminotransferase), AST (aspartate aminotransferase), and TBIL (total bilirubin) in plasma were determined. An increase in liver markers in this model indicates the potential of that compound to cause liver toxicity in humans at lower doses following longer exposures.

**Table 2: Plasma levels of liver markers of mice 24 h after treatment with either vehicle or test compounds No.142, No.171 or fingolimod.**

| **Compound No.** | | **ALT (IU/L)** | **AST (IU/L)** | **TBil (mg/dl)** |
|---|---|---|---|---|
| Vehicle | | 51 ± 9 | 64 ± 9 | 0.5 ± 0.1 |
| 142 (100 mg/kg) | | 45 ± 5 | 87 ± 9 | 0.3 ± 0.1 |
| Vehicle | | 43 ± 6 | 65 ± 5 | 0.5 ± 0.04 |
| 171 (100 mg/kg) | | 42 ± 13 | 66 ± 7 | 0.5 ± 0.04 |
| Vehicle | | 62 ± 4 | 100 ± 24 | 0.17 ± 0.04 |
| Fingolimod | | | | |
| | 30 mg/kg | 59 ± 62 | 354 ± 70* | 0.12 ± 0.01 |
| | 100 mg/kg | 355 ± 60** | 589 ± 73*** | 0.16 ± 0.02 |

| | | | | |
|---|---|---|---|---|
| *One-way Anova with Dunet's post test, *p<0.05, **p<0.01, ***p<0.005* | | | | |

As it is shown in Table 2, fingolimod-treated mice showed a dose-response increase in AST and ALT levels compared to its corresponding vehicle-treated group, clearly indicating a high hepatotoxicity at the two doses studied. By contrast, the S1P1 agonist compounds according to the present invention did not cause a significant increase in any of the parameters measured.

The lack of hepatotoxicity observed with Compounds 142 and 171 in this model suggests that the risk of liver toxicity is lower than that of fingolimod. This has implications in monotherapy, when compounds are administered as stand-alone treatments, but the most profound implications are in combination with other potential hepatotoxic drugs.

### Example 3: Half life in rats after intravenous administration.

Rats were administered with 1 mg/kg of test compounds by intraveneous route to Wistar rats. Blood samples were collected from retroorbital plexus at several time points after administration and plasma levels of compounds were quantified by MS-HPLC.

In samples of rats treated with fingolimod, both the parental compound (fingolimod) and the active metabolite (fingolimod-phosphate) were determined.

**Table3: Pharmacokinetic parameters of fingolimod, Compounds No. 142 and No. 171 in rats following i.v. administration of 1 mg/kg.**

| Compound No. | **Cmax(ng/ml) Plasma** | **Tmax** **(h)** | **AUC 0-24** **(ng.h/ml)** | **Half life** **t_{1/2} (h)** |
|---|---|---|---|---|
| Fingolimod* | 50.5 | 0.1 | 279 | 26.1 |
| Fingolimod-P | 92.1 | 1 | 1329 | 24.4 |
| 142 | 824 | 0.1 | 2627 | 3.5 |
| 171 | 986 | 0.1 | 6227 | 8.2 |

| | | | | |
|---|---|---|---|---|
| *Animals were administered with the parental compound, fingolimod, and values from both fingolimod and its in vivo generated active metabolite, fingolimod-phosphate, have been measured. | | | | |

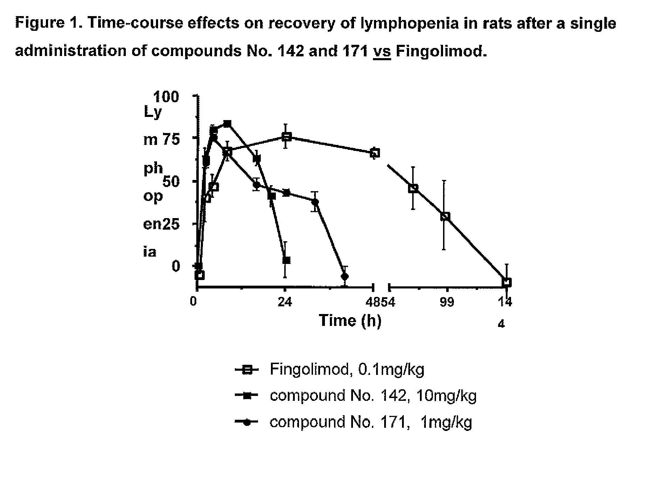

The advantage of compound No. 142 or compound No. 171 is that they have a shorter half life than fingolimod and this is translated into a faster recovery of lymphopenia (Figure 1). In the event of an unexpected toxicity leading to treatment discontinnuation, the clearance of the compound and thus the dissappearance of the side effect will be faster with compounds of the present invention than that with Fingolimod.

### Example 3. Recovery of lymphopenia in rats after a single oral administration

Test compounds were administered by oral route to Wistar rats at a dose of each compound that caused maximal lymphopenia. At several times after administration, animals were anestesized and blood extracted from retroorbital plexus and collected into heparinized tubes. Total lymphocyte numbers were determined using a Sysmex K-800 haemocytometer.

In agreement with their shorter half lives (Table 3), animals treated with compounds Nos. 142 and 171 recover lymphocytes in blood between 24 and 40h after administration. However, 144h are needed for the same effect to occur with fingolimod. Thus, patients treated with compounds of the present invention will recover their immunocompetent status in a more shorter time than in the case of fingolimod when the treatment has to be discontinued due to an opportunistic infection.

### BRIEF DESCRIPTION OF THE DRAWING

Figure 1 shows the time-course effect on recovery of lymphopenia in rats after a single oral administration of Fingolimod and of compounds No. 142 and 171.

## Claims

1. A S1P1 receptor agonist of formula (I), or a pharmaceutically acceptable salt, or solvate, or N-oxide, or stereoisomer or deuterated derivate thereof, for use in the treatment of Crohn's disease: 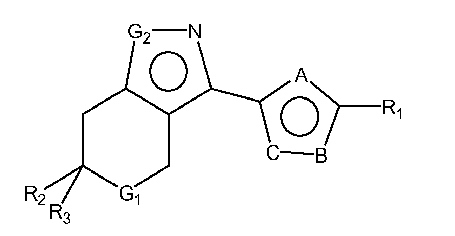 Formula (I)
wherein
either (i) A is selected from the group consisting of -N-, -O- and -S-; B and C are independently selected from the group consisting of -N- and -O-, with the proviso that two of A, B and C are nitrogen atoms, or (ii) two of A, B and C are -N- and one of A, B and C is -NH-;
G¹ is selected from the group consisting of -CH₂-, -NH- and -O-;
G² is selected from the group consisting of -NR⁴- and -O-;
R¹ represents:
➢ a 8 to 10 membered bicyclic N-containing heteroaryl group optionally substituted with a C₁₋₄ carboxyalkyl group or a C₁₋₄ aminoalkyl group,
➢ a pyridyl group optionally substituted with one or more substituents selected from hydroxy groups, C₁₋₄ alkyl groups, C₁₋₄ carboxyalkyl groups, C₁₋₄ haloalkyl groups, C₁₋₄ alkoxy groups, amino groups, C₁₋₄ aminoalkyl groups and C₁₋₄ aminoalkoxy groups,
➢ a pyridone group substituted with one or more C₁₋₄ alkyl groups; C₁₋₄ haloalkyl groups or C₁₋₄ aminoalkyl groups, or
➢ a group of formula: 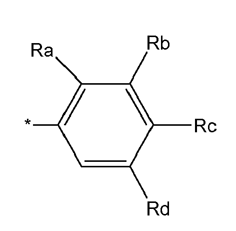
wherein:
• R^{a} represents a hydrogen atom, a halogen atom, a C₁₋₄ alkyl group, C₃₋₄ cycloalkyl group or a -CF₃ group;
• R^{b} represents a hydrogen atom, a halogen atom, a C₁₋₄ alkyl group, a -CF₃ group or a C₁₋₄ alkoxy group;
• R^{d} represents a hydrogen atom, a C₁₋₄ alkyl group or a C₁₋₄ alkoxy group;
• R^{c} represents:
○ a hydrogen atom, a C₁₋₄ hydroxyalkyl group, a C₁₋₄ aminoalkyl group which is optionally substituted with one or more substituents selected from halogen atoms, hydroxy groups and -CF₃ groups;
○ a 4 to 6-membered saturated N-containing heterocyclic ring optionally substituted with a C₁₋₂ carboxyalkyl group;
○ -(CH₂)(₀₋₄)-C(O)OR', -(CH₂)(₀₋₄)-C(O)NR'R", -(CH₂)(₀₋₄)-NHC(O)R", -S(O)₂NR'R", -O-(CH₂)(₂₋₄)NR'R", -O-(CH₂)(₁₋₄)C(O)OR", -O-(CH₂)(₁₋₄)-C(O)NR'R", -(CH₂)(₀₋₄)-NR'R", -(CH₂)(₀-₄)-CONHS(O)₂R', -(CH₂)(₀₋₄)-NHS(O)₂R' or -(CH2)₍₀₋₃₎-NH-(CH₂)₍₁₋₃₎-(NH)₍₀₋₁₎S(O)₂R' wherein,
■ R' represents a hydrogen atom or a C₁₋₄ alkyl group,
■ R" represents a hydrogen atom, a C₁₋₄ alkyl group, a C₃₋₄ cycloalkyl group, a C₁₋₄ carboxyalkyl group, a C₁₋₄ haloalkyl group, a C₁₋₄ hydroxyalkyl group or a 6 membered, saturated N-containing heterocyclic ring, or
■ R' and R" together with the nitrogen atom to which they are attached form a 4 to 6 membered heterocyclic group which contains, as heteroatoms, one N atom and, optionally, one further atom selected from N and O, and which is optionally substituted with a carboxy or a C₁₋₄ carboxyalkyl group,
or R^{c} together with R^{d} form a C₅₋₆ cycloalkyl group optionally substituted by a -NHR^{f} group, wherein R^{f} is selected from the group consisting of a hydrogen atom and a carboxymethyl group;
R² and R³ are independently selected from the group consisting of hydrogen atoms, halogen atoms and C₁₋₄ alkyl groups; and
R⁴ is selected from the group consisting of a hydrogen atom, a phenyl group, a C₃₋₄ cycloalkyl-C₁₋₄ alkyl group, C₁₋₄ aminoalkyl group, C₁₋₄ haloalkyl group and a linear or branched C₁₋₄ alkyl group which is optionally substituted by a phenyl or a pyridyl group.

2. The S1P1 receptor agonist according to claim 1, wherein A is selected from the group consisting of -N- and -O-.

3. The S1P1 receptor agonist according to claim 2, wherein A represents -N-.

4. The S1P1 receptor agonist according to any one of claims 1 to 3, wherein both A and B represent -N- and C represents -O-.

5. The S1P1 receptor agonist according to any one of the preceding claims, wherein G¹ represents a -CH₂- or a -O- group.

6. The S1P1 receptor agonist according to claim 5, wherein G¹ represents a -CH₂-group.

7. The S1P1 receptor agonist according to any one of the preceding claims, wherein R² and R³ are independently selected from the group consisting of hydrogen atoms, fluorine atoms and methyl groups.

8. The S1P1 receptor agonist according to claim 7, wherein both R² and R³ are methyl groups.

9. The S1P1 receptor agonist according to any one of claims 1 to 8, wherein R⁴ is selected from the group consisting of a C₃₋₄ cycloalkyl-C₁₋₄ alkyl group, C₁₋₄ haloalkyl group and a linear or branched unsubstituted C₁₋₄ alkyl group.

10. The S1P1 receptor agonist according to claim 9, wherein G² represents-NR⁴-, and wherein R⁴ is selected from the group consisting of a methyl group, ethyl group, t-butyl group, cyclopropylmethyl group and 2,2,2-trifluoroethyl group.

11. The S1P1 receptor agonist according to claim 10, wherein R⁴ represents a methyl or an ethyl group.

12. The S1P1 receptor agonist according to any one of the preceding claims, wherein R¹ represents:
➢ a pyridyl group substituted with one, two or three substituents selected from hydroxy groups and C₁₋₄ alkyl groups;
➢ a pyridone group substituted with one or two C₁₋₂ alkyl groups; or
➢ a group of formula: 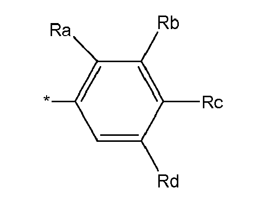 wherein:
• R^{a} represents a hydrogen atom or a C₁₋₄ alkyl group;
• R^{b} represents a hydrogen atom or C₁₋₄ alkyl group;
• R^{d} represents a hydrogen atom or a C₁₋₄ alkyl group;
• R^{c} represents:
○ a C₁₋₄ hydroxyalkyl group or a C₁₋₄ aminoalkyl substitued by one or more halogen atoms;
○ -(CH₂)₍₂₋₃₎-C(O)OR', -(CH₂)₍₀₋₂₎-C(O)NR'R", -O-(CH₂)(₂₋₃)NR'R", -(CH₂)₍₂₋₃₎-NHC(O)R", -S(O)₂NR'R", -(CH₂)₍₀₋₃)-NR'R" or -(CH₂)₍₁₋₂₎-CONHS(O)₂R' wherein,
■ R' represents a hydrogen atom or a methyl group,
■ R" represents a hydrogen atom, a C₁₋₄ alkyl group, C₁₋₄ carboxyalkyl group, C₁₋₄ haloalkyl group or a C₁₋₄ hydroxyalkyl group, or
■ R' and R" together with the nitrogen atom to which they are attached from a 4 to 6 membered heterocyclic group which contains, as heteroatoms, one N atom and, optionally, one further N atom, and which is optionally substituted with a carboxy or a C₁₋₂ carboxyalkyl group,
or R^{c} together with R^{d} form a cyclohexyl group substituted with a carboxymethylamino group.

13. The S1P1 receptor agonist according to claim 12, wherein R¹ represents:
➢ a pyridyl group substituted with two or three substituents selected from hydroxy groups, methyl and ethyl groups, or
➢ a group of formula: 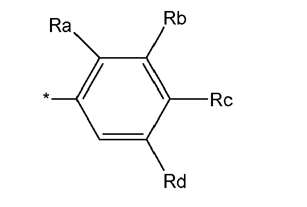 wherein:
• R^{a} represents a hydrogen atom or a methyl group;
• R^{b} represents a hydrogen atom, a methyl group;
• R^{d} represents a hydrogen atom or a methyl group;
• R^{c} represents: -(CH₂)₍₂₋₃₎-C(O)OR', -(CH₂)₂-C(O)NR'R" or -(CH₂)₍₂₋₃₎-NR'R", wherein
o R' represents a hydrogen atom;
o R" represents a hydrogen atom, a C₁₋₂ carboxyalkyl group, a C₁₋₄ haloalkyl group or a C₁₋₂ hydroxyalkyl group; or
o R' and R" together with the nitrogen atom to which they are attached form a 4 membered saturated heterocyclic group, which contains as heteroatom, one nitrogen atom and which is substituted with a carboxy group.

14. The S1P1 receptor agonist according to claim 13, wherein R¹ represents a group of formula: 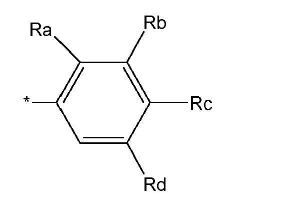 wherein:
o R^{a} represents a hydrogen atom;
o both R^{b} and R^{d} represent methyl groups; and
o R^{c} represents -(CH₂)₍₂₋₃₎-C(O)OH or -(CH₂)₍₂₋₃₎-NHR", wherein R" is selected from a hydrogen atom, a C₁₋₂ carboxyalkyl group, and C₁₋₂ hydroxyalkyl group.

15. The S1 P1 receptor agonist according to any one of the preceding claims wherein:
G¹ represents a -CH₂- group,
G² represents a -NR₄- group, wherein R⁴ represents a methyl or ethyl group,
both R² and R³ represent a methyl group, and
R¹ represents:
➢ a pyridyl group substituted with two or three substituents selected from hydroxy groups, methyl or ethyl groups, or
➢ a group of formula: 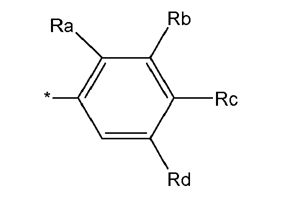 wherein:
• R^{a} represents a hydrogen atom or a methyl group;
• R^{b} represents a hydrogen atom, a methyl group,
• R^{d} represents a hydrogen atom or a methyl group,
• R^{c} represents: -(CH₂)(₂₋₃)-C(O)OR', -(CH₂)₂-C(O)NR'R" or -(CH₂)(₂-₃)-NR'R", wherein:
o R' represents a hydrogen atom;
o R" represents a hydrogen atom, a C₁₋₂ carboxyalkyl group, a C₁₋₄ haloalkyl group or a C₁-₂ hydroxyalkyl group, or
o R' and R" together with the nitrogen atom to which they are attached form a 4 membered saturated heterocyclic group, which contains as heteroatom, one nitrogen atom and which is substituted with a carboxy group.

16. The S1P1 receptor agonist according to claim 15, wherein R¹ represents a group of formula: 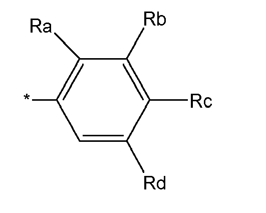 wherein
o R^{a} represents a hydrogen atom;
o both R^{b} and R^{d} represents a methyl group and
o R^{c} represents -(CH₂)₍₂₋₃₎-C(O)OH or -(CH₂)₍₂₋₃₎-NHR", wherein R" is selected from a hydrogen atom, a C₁₋₂ carboxyalkyl group and C₁₋₂ hydroxyalkyl group.

17. The S1 P1 receptor agonist of according to claim 1, wherein R¹ represents:
➢ an imidazo[1,2-a]pyridyl group or a 3H-pyrrolo[2,3-b]pyridyl group which are optionally substituted with a carboxyethyl group;
➢ a pyridyl group optionally substituted with one or more substituents selected from hydroxy groups, methyl groups, ethyl groups, carboxyethyl groups, -CF₃ groups, methoxy groups and amino groups
➢ a pyridone group substituted with one or more substituents selected from methyl and ethyl groups; or
➢ a group of formula: 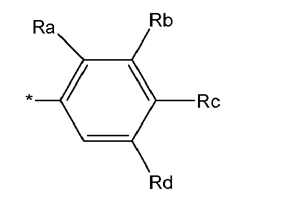 wherein:
• R^{a} represents a hydrogen atom, a methyl group, cyclopropyl group or a CF₃ group;
• R^{b} represents a hydrogen atom, a chlorine atom or a methyl group;
• R^{d} represents a hydrogen atom or a methyl group;
• R^{c} represents:
o a C₁₋₄ hydroxyalkyl group or a C₁₋₄ aminoalkyl group substituted with one or more substituents selected from fluorine atoms and hydroxy groups;
o a 4 to 6-membered saturated N-containing heterocyclic ring which is optionally substituted with a C₁₋₂ carboxyalkyl group
o -(CH₂)₍₀₋₄₎-C(O)OR', -(CH₂)₍₀₋₄₎-C(O)NR'R", -(CH₂)₍₀₋₄₎-NHC(O)R", -S(O)₂NR'R", -O-(CH₂)₍₂₋₄₎NR'R", -O-(CH₂)₍₁₋₄₎C(O)OR", -O-(CH₂)₍₁₋₄₎-C(O)NR'R" -(CH₂)₍₀₋₄₎-NR'R", -(CH₂)₍₀₋₄₎-CONHS(O)₂R' -(CH₂)₍₀₋₄₎-NHS(O)2R' or -(CH2)₍₀₋₃₎-NH-(CH₂)₍₁₋₃₎-(NH)₍₀₋₁₎S(O)₂R' wherein
o R' represents a hydrogen atom or a methyl group,
o R" represents a hydrogen atom, a methyl group, a cyclopropyl group, a piperidyl group, a C₁₋₂ carboxyalkyl group, a CF₃ group, C₁₋₄ hydroxyalkyl group, or
o R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₄ carboxyalkyl group,
or R^{c} together with R^{d} form a cyclohexyl group optionally substituted by a -NHR^{f} group, wherein R^{f} is selected from the group consisting of a hydrogen atom and a carboxymethyl group;
R² and R³ are independently selected from the group consisting of hydrogen atoms, fluorine atoms and methyl groups; and
R⁴ is selected from the group consisting of a hydrogen atom, a phenyl group, a C₃₋₄ cycloalkyl-C₁₋₂ alkyl group, C₁₋₂ aminoalkyl group, C₁₋₂ haloalkyl group or R⁴ represents a linear or branched C₁₋₄ alkyl group which is optionally substituted by a phenyl group or a pyridyl group.

18. The S1P1 receptor agonist according to any one of claims 1 to 17, wherein R¹ represents a group of formula: 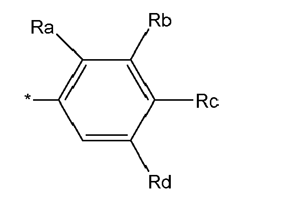 wherein:
• R^{a} represents a hydrogen atom,
• R^{b} represents a methyl group or a CF₃ group,
• R^{d} represents a hydrogen atom or a methyl group;
• R^{c} represents a -(CH₂)₍₀₋₄₎-C(O)NR'R", -(CH₂)₍₀₋₄₎-NHC(O)R" or -(CH₂)₍₀₋₄₎-NR'R", wherein
o R' represents a hydrogen atom or a methyl group,
o R" represents a hydrogen atom, a methyl group, a C₁-₂ carboxyalkyl group or a C₁₋₄ hydroxyalkyl group, or
o R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₄ carboxyalkyl group

19. The S1P1 receptor agonist according to claim 18, wherein R^{c} represents a -(CH₂)₍₂₋₃₎-NR'R", wherein R' and R" together with the nitrogen atom to which they are attached from a 4 to 6 membered heterocyclic group which contains, as heteroatoms, one N atom, and which is substituted with a carboxy or a C₁₋₂ carboxyalkyl group.

20. The S1P1 receptor agonist according to claim 1, which is a compound of general formula (I'), or a pharmaceutically acceptable salt or N-oxide thereof: 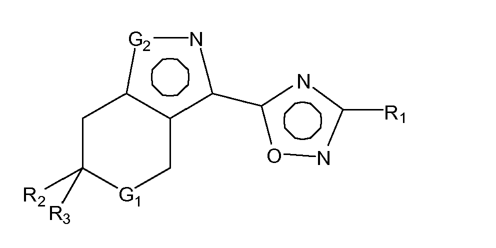 Formula (I')
wherein,
G¹ is selected from the group consisting of -CH₂-, and -O-;
G² is selected from the group consisting of -NR⁴- and -O-;
R¹ represents:
➢ a pyrrolopyridyl group, which is unsubstituted or substituted with a C₁₋₂ carboxyalkyl group;
➢ a pyridyl group optionally substituted with 1, 2 or 3 substituents selected from hydroxy groups, C₁₋₂ alkyl groups, C₁₋₂ carboxyalkyl groups, C₁₋₂ haloalkyl groups, C₁₋₂ alkoxy groups, and amino groups;
➢ a pyridone group substituted with 1, 2 or 3 C₁₋₂ alkyl groups; or
➢ a group of formula: 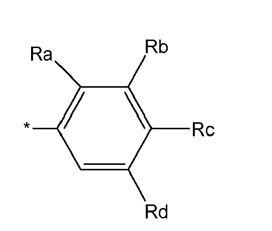
wherein:
• R^{a} represents a hydrogen atom, a C₁₋₂ alkyl group, a cyclopropyl group or a -CF₃ group;
• R^{b} represents a hydrogen atom, a chlorine atom, or a C₁-₂ alkyl group;
• R^{d} represents a hydrogen atom, or a C₁₋₂ alkyl group;
• R^{c} represents:
o a C₁₋₃ hydroxyalkyl group;
o a carboxyethylpiperazine group;
o -(CH₂)₍₀₋₂₎-C(O)OR', -(CH₂)₍₀₋₂₎-C(O)NR'R", -S(O)₂NR'R", or -(CH₂)₍₀₋₄₎-NR'R", wherein,
■ R' represents a hydrogen atom,
■ R" represents a hydrogen atom, a C₁₋₂ alkyl group, a cyclopropyl group, a C₁₋₂ carboxyalkyl group, a C₁₋₂ haloalkyl group, a C₁₋₂ hydroxyalkyl group or a piperidyl group, or
■ R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₂ carboxyalkyl group,
or R^{c} together with R^{d} forms a cyclohexyl group substituted by a -NHR^{f} group, wherein R^{f} is a carboxymethyl group;
R² and R³ are independently selected from the group consisting of hydrogen atoms, fluorine atoms and C₁₋₂ alkyl groups; and
R⁴ is selected from the group consisting of hydrogen atoms, phenyl groups, cyclopropyl-C₁₋₂ alkyl groups, C₁₋₂ aminoalkyl groups, C₁₋₂ haloalkyl groups and linear or branched C₁₋₄ alkyl groups which are optionally substituted by a phenyl or a pyridyl group.

21. The S1P1 receptor agonist according to claim 1, which is one of:
4-(5-(6,6-Dimethyl-4,5,6,7-tetrahydro-2H-indazol-3-yl)-1,2,4-oxadiazol-3-yl)benzenesulfonamide,
(4-(5-(6,6-Dimethyl-4,5,6,7-tetrahydro-2H-indazol-3-yl)-1,2,4-oxadiazol-3-yl)phenyl)methanol,
(4-(5-(1,6,6-Trimethyl-4,5,6,7-tetrahydro-1H-indazol-3-yl)-1,2,4-oxadiazol-3-yl)phenyl)methanol,
4-(5-(1,6,6-Trimethyl-4,5,6,7-tetrahydro-1H-indazol-3-yl)-1,2,4-oxadiazol-3-yl)benzenesulfonamide,
4-(5-(1-Ethyl-6,6-dimethyl-4,5,6,7-tetrahydro-1H-indazol-3-yl)-1,2,4-oxadiazol-3-yl)benzenesulfonamide,
5-(1-Ethyl-6,6-dimethyl-4,5,6,7-tetrahydro-1H-indazol-3-yl)-3-(pyridin-4-yl)-1,2,4-oxadiazole,
3-(4-(5-(1-Ethyl-6,6-dimethyl-4,5,6,7-tetrahydro-1H-indazol-3-yl)-1,2,4-oxadiazol-3-yl)-2,6-dimethylphenyl)propanoic acid,
3-(4-(4-(5-(1-Ethyl-6,6-dimethyl-4,5,6,7-tetrahydro-1H-indazol-3-yl)-1,2,4-oxadiazol-3-yl)phenyl)piperazin-1-yl)propanoic acid,
4-(5-(1-Ethyl-6,6-dimethyl-4,5,6,7-tetrahydro-1H-indazol-3-yl)-1,2,4-oxadiazol-3-yl)benzoic acid,
4-(5-(1-Ethyl-6,6-dimethyl-4,5,6,7-tetrahydro-1H-indazol-3-yl)-1,2,4-oxadiazol-3-yl)benzamide,
5-(1-Ethyl-6,6-dimethyl-4,5,6,7-tetrahydro-1H-indazol-3-yl)-3-(4-(piperazin-1-yl)phenyl)-1,2,4-oxadiazole,
2-(4-(5-(1-Ethyl-6,6-dimethyl-4,5,6,7-tetrahydro-1H-indazol-3-yl)-1,2,4-oxadiazol-3-yl)phenyl)acetic acid,
1-(4-(5-(1-Ethyl-6,6-dimethyl-4,5,6,7-tetrahydro-1H-indazol-3-yl)-1,2,4-oxadiazol-3-yl)benzyl)azetidine-3-carboxylic acid,
2-(4-(5-(1-Ethyl-6,6-dimethyl-4,5,6,7-tetrahydro-1H-indazol-3-yl)-1,2,4-oxadiazol-3-yl)phenyl)acetamide,
4-(5-(1-Ethyl-6,6-dimethyl-4,5,6,7-tetrahydro-1H-indazol-3-yl)-1,2,4-oxadiazol-3-yl)pyridine 1-oxide,
N-(4-(5-(1-Ethyl-6,6-dimethyl-4,5,6,7-tetrahydro-1H-indazol-3-yl)-1,2,4-oxadiazol-3-yl)phenyl)piperidin-4-amine,
5-(1-Ethyl-6,6-dimethyl-4,5,6,7-tetrahydro-1H-indazol-3-yl)-3-(6-methoxypyridin-3-yl)-1,2,4-oxadiazole,
5-(5-(1-Ethyl-6,6-dimethyl-4,5,6,7-tetrahydro-1H-indazol-3-yl)-1,2,4-oxadiazol-3-yl)pyridin-2-ol,
4-(5-(1-Ethyl-6,6-dimethyl-4,5,6,7-tetrahydro-1H-indazol-3-yl)-1,2,4-oxadiazol-3-yl)pyridin-2-ol,
3-(4-(5-(1-Ethyl-6,6-dimethyl-4,5,6,7-tetrahydro-1H-indazol-3-yl)-1,2,4-oxadiazol-3-yl)-2,6-dimethylphenyl)propanamide,
4-(5-(1-Benzyl-6,6-dimethyl-4,5,6,7-tetrahydro-1H-indazol-3-yl)-1,2,4-oxadiazol-3-yl)benzamide,
4-(5-(1-tert-Butyl-6,6-dimethyl-4,5,6,7-tetrahydro-1H-indazol-3-yl)-1,2,4-oxadiazol-3-yl)benzamide,
5-(1-Ethyl-6,6-dimethyl-4,5,6,7-tetrahydro-1H-indazol-3-yl)-3-(2-methoxypyridin-4-yl)-1,2,4-oxadiazole,
4-(5-(1-Ethyl-6,6-dimethyl-4,5,6,7-tetrahydro-1H-indazol-3-yl)-1,2,4-oxadiazol-3-yl)-1-methylpyridin-2(1H)-one,
1-(4-(5-(1-Ethyl-6,6-dimethyl-4,5,6,7-tetrahydro-1H-indazol-3-yl)-1,2,4-oxadiazol-3-yl)phenyl)piperidine-4-carboxylic acid,
(4-(5-(1-Ethyl-6,6-dimethyl-4,5,6,7-tetrahydro-1H-indazol-3-yl)-1,2,4-oxadiazol-3-yl)phenyl)methanol,
4-(5-(6,6-Dimethyl-1-phenyl-4,5,6,7-tetrahydro-1H-indazol-3-yl)-1,2,4-oxadiazol-3-yl)benzamide,
3-Ethyl-5-(5-(1-ethyl-6,6-dimethyl-4,5,6,7-tetrahydro-1H-indazol-3-yl)-1,2,4-oxadiazol-3-yl)-6-methylpyridin-2-ol,
5-(5-(1-Ethyl-6,6-dimethyl-4,5,6,7-tetrahydro-1H-indazol-3-yl)-1,2,4-oxadiazol-3-yl)-3-methylpyridin-2-ol,
4-(5-(1-Ethyl-6,6-dimethyl-4,5,6,7-tetrahydro-1H-indazol-3-yl)-1,2,4-oxadiazol-3-yl)-3-methylbenzamide,
4-(4-(5-(1-Ethyl-6,6-dimethyl-4,5,6,7-tetrahydro-1H-indazol-3-yl)-1,2,4-oxadiazol-3-yl)benzyl)morpholine,
3-Ethyl-5-(5-(1-ethyl-6,6-dimethyl-4,5,6,7-tetrahydro-1H-indazol-3-yl)-1,2,4-oxadiazol-3-yl)-1,6-dimethylpyridin-2(1 H)-one,
5-(5-(1-Ethyl-6,6-dimethyl-4,5,6,7-tetrahydro-1H-indazol-3-yl)-1,2,4-oxadiazol-3-yl)-1,3-dimethylpyridin-2(1H)-one,
N-Cyclopropyl-4-(5-(1-ethyl-6,6-dimethyl-4,5,6,7-tetrahydro-1H-indazol-3-yl)-1,2,4-oxadiazol-3-yl)benzamide,
1-(4-(5-(1-Ethyl-6,6-dimethyl-4,5,6,7-tetrahydro-1H-indazol-3-yl)-1,2,4-oxadiazol-3-yl)phenyl)-N-methylmethanamine,
5-(1-Ethyl-6,6-dimethyl-4,5,6,7-tetrahydro-1H-indazol-3-yl)-3-(3-methylpyridin-4-yl)-1,2,4-oxadiazole,
4-(5-(6,6-Dimethyl-1-(2,2,2-trifluoroethyl)-4,5,6,7-tetrahydro-1H-indazol-3-yl)-1,2,4-oxadiazol-3-yl)benzam ide,
(4-(5-(1-Ethyl-6,6-dimethyl-4,5,6,7-tetrahydro-1H-indazol-3-yl)-1,2,4-oxadiazol-3-yl)phenyl)methanamine,
5-(1-Ethyl-6,6-dimethyl-4,5,6,7-tetrahydro-1H-indazol-3-yl)-3-(3-methylpyridin-2-yl)-1,2,4-oxadiazole,
2-(4-(5-(1-Ethyl-6,6-dimethyl-4,5,6,7-tetrahydro-1H-indazol-3-yl)-1,2,4-oxadiazol-3-yl)-2,6-dimethylphenyl)ethanamine,
5-(5-(1-Ethyl-6,6-dimethyl-4,5,6,7-tetrahydro-1H-indazol-3-yl)-1,2,4-oxadiazol-3-yl)-4-methylpyridin-2-ol,
3-(4-(5-(1-Ethyl-6,6-dimethyl-4,5,6,7-tetrahydro-1H-indazol-3-yl)-1,2,4-oxadiazol-3-yl)-3-methylphenyl)propanoic acid,
5-(5-(1-Ethyl-6,6-dimethyl-4,5,6,7-tetrahydro-1H-indazol-3-yl)-1,2,4-oxadiazol-3-yl)-6-methylpyridin-2-ol,
5-(1-Ethyl-6,6-dimethyl-4,5,6,7-tetrahydro-1H-indazol-3-yl)-3-(4-methylpyridin-3-yl)-1,2,4-oxadiazole,
5-(1-Ethyl-6,6-dimethyl-4,5,6,7-tetrahydro-1H-indazol-3-yl)-3-(4-(trifluoromethyl)-pyridin-3-yl)-1,2,4-oxadiazole,
5-(1-Ethyl-6,6-dimethyl-4,5,6,7-tetrahydro-1H-indazol-3-yl)-3-(imidazo[1,2-a]pyridin-6-yl)-1,2,4-oxadiazole,
3-(4-(5-(1-Ethyl-6,6-dimethyl-4,5,6,7-tetrahydro-1H-indazol-3-yl)-1,2,4-oxadiazol-3-yl)-3-methylphenyl)propanamide,
2-(4-(5-(1-Ethyl-6,6-dimethyl-4,5,6,7-tetrahydro-1H-indazol-3-yl)-1,2,4-oxadiazol-3-yl)-3-methylphenyl)ethanamine,
3-(4-(5-(1-Ethyl-6,6-dimethyl-4,5,6,7-tetrahydro-1H-indazol-3-yl)-1,2,4-oxadiazol-3-yl)-2,6-dimethylphenyl)propan-1-amine,
4-(5-(1-Ethyl-6,6-dimethyl-4,5,6,7-tetrahydro-1H-indazol-3-yl)-1,2,4-oxadiazol-3-yl)-3-(trifluoromethyl)benzoic acid,
4-(5-(1-Ethyl-6,6-dimethyl-4,5,6,7-tetrahydro-1H-indazol-3-yl)-1,2,4-oxadiazol-3-yl)-3-(trifluoromethyl)benzamide,
5-(1-Ethyl-6,6-dimethyl-4,5,6,7-tetrahydro-1H-indazol-3-yl)-3-(2-methylpyridin-3-yl)-1,2,4-oxadiazole,
5-(5-(1-Ethyl-6,6-dimethyl-4,5,6,7-tetrahydro-1H-indazol-3-yl)-1,2,4-oxadiazol-3-yl)-6-(trifluoromethyl)pyridin-2-amine,
3-Cyclopropyl-4-[5-(1-ethyl-6,6-dimethyl-4,5,6,7-tetrahydro-1H-indazol-3-yl)-1,2,4-oxadiazol-3-yl]benzamide,
5-[5-(1-Ethyl-6,6-dimethyl-4,5,6,7-tetrahydro-1H-indazol-3-yl)-1,2,4-oxadiazol-3-yl]-6-(trifluoromethyl)pyridin-2-ol,
5-(5-(6,6-Dimethyl-1-(2,2,2-trifluoroethyl)-4,5,6,7-tetrahydro-1H-indazol-3-yl)-1,2,4-oxadiazol-3-yl)-3-ethyl-6-methylpyridin-2-ol,
3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propanoic acid,
3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propanamide,
3-Ethyl-5-[5-(1-ethyl-6,6-dimethyl-1,4,6,7-tetrahydropyrano[4,3-c]pyrazol-3-yl)-1,2,4-oxadiazol-3-yl]-6-methylpyridin-2-ol,
3-(4-(5-(1-Ethyl-6,6-dimethyl-4,5,6,7-tetrahydro-1H-indazol-3-yl)-1,2,4-oxadiazol-3-yl)-3-methylphenyl)propan-1-amine,
3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propan-1-amine,
6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-amine,
2-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)ethanamine,
3-(2,6-Dimethyl-4-(5-(1,6,6-trimethyl-4,5,6,7-tetrahydro-1H-indazol-3-yl)-1,2,4-oxadiazol-3-yl)phenyl)propanoic acid,
3-(2,6-Dimethyl-4-(5-(1,6,6-trimethyl-4,5,6,7-tetrahydro-1H-indazol-3-yl)-1,2,4-oxadiazol-3-yl)phenyl)propanamide,
3-(4-(5-(6,6-Dimethyl-4,5,6,7-tetrahydrobenzo[d]isoxazol-3-yl)-1,2,4-oxadiazol-3-yl)-2,6-dimethylphenyl)propanoic acid,
3-(4-(5-(6,6-Dimethyl-4,5,6,7-tetrahydrobenzo[d]isoxazol-3-yl)-1,2,4-oxadiazol-3-yl)-2,6-dimethylphenyl)propanamide,
2-(6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-ylamino)ethanoic acid,
3-(2,6-Dimethyl-4-(5-(1,6,6-trimethyl-4,5,6,7-tetrahydro-1H-indazol-3-yl)-1,2,4-oxadiazol-3-yl)phenyl)propan-1-amine,
3-(4-(5-(6,6-Dimethyl-4,5,6,7-tetrahydrobenzo[d]isoxazol-3-yl)-1,2,4-oxadiazol-3-yl)-2,6-dimethylphenyl)propan-1-amine,
2-(2,6-Dimethyl-4-(5-(1,6,6-trimethyl-4,5,6,7-tetrahydro-1H-indazol-3-yl)-1,2,4-oxadiazol-3-yl)phenyl)ethanamine,
2-(4-(5-(6,6-Dimethyl-4,5,6,7-tetrahydrobenzo[d]isoxazol-3-yl)-1,2,4-oxadiazol-3-yl)-2,6-dimethylphenyl)ethanamine,
5-(5-(6,6-Dimethyl-1-(pyridin-3-ylmethyl)-4,5,6,7-tetrahydro-1H-indazol-3-yl)-1,2,4-oxadiazol-3-yl)-3-ethyl-6-methylpyridin-2(1H)-one,
2-(Dimethyl-4,5,6,7-tetrahydrobenzo[d]isoxazol-3-yl)-1,2,4-oxadiazol-3-yl)-2,6-dimethylphenethylamino)ethanoic acid,
2-(3-(4-(5-(6,6-Dimethyl-4,5,6,7-tetrahydrobenzo[d]isoxazol-3-yl)-1,2,4-oxadiazol-3-yl)-2,6-dimethylphenyl)propylamino)ethanoic acid,
3-(Dimethyl-4,5,6,7-tetrahydrobenzo[d]isoxazol-3-yl)-1,2,4-oxadiazol-3-yl)-2,6-dimethylphenethylamino)propanoic acid,
3-Ethyl-5-(5-(1-ethyl-4,5,6,7-tetrahydro-1H-indazol-3-yl)-1,2,4-oxadiazol-3-yl)-6-methylpyridin-2(1H)-one,
3-Ethyl-6-methyl-5-(5-(1,6,6-trimethyl-4,5,6,7-tetrahydro-1H-indazol-3-yl)-1,2,4-oxadiazol-3-yl)pyridin-2-ol,
5-(5-(6,6-Dimethyl-4,5,6,7-tetrahydrobenzo[d]isoxazol-3-yl)-1,2,4-oxadiazol-3-yl)-3-ethyl-6-methylpyridin-2(1H)-one,
5-(5-(1-(2-Aminoethyl)-6,6-dimethyl-4,5,6,7-tetrahydro-1H-indazol-3-yl)-1,2,4-oxadiazol-3-yl)-3-ethyl-6-methylpyridin-2-ol,
3-Ethyl-5-(5-(1-ethyl-6,6-dimethyl-4,5,6,7-tetrahydro-1H-indazol-3-yl)-1,2,4-oxadiazol-3-yl)-6-methylpyridin-2-amine,
2-(4-(5-(6,6-Dimethyl-4,5,6,7-tetrahydrobenzo[d]isoxazol-3-yl)-1,2,4-oxadiazol-3-yl)-2,6-dimethylphenyl)-N,N-dimethylethanamine,
3-(4-(4-(5-(6,6-Dimethyl-4,5,6,7-tetrahydrobenzo[d]isoxazol-3-yl)-1,2,4-oxadiazol-3-yl)phenyl)piperazin-1-yl)propanoic acid,
3-Ethyl-5-(5-(1-ethyl-6,6-difluoro-4,5,6,7-tetrahydro-1H-indazol-3-yl)-1,2,4-oxadiazol-3-yl)-6-methylpyridin-2(1H)-one,
3-(3-Ethyl-5-(5-(1-ethyl-6,6-dimethyl-4,5,6,7-tetrahydro-1H-indazol-3-yl)-1,2,4-oxadiazol-3-yl)-6-methylpyridin-2-yl)propanoic acid,
5-(5-(1-(Cyclopropylmethyl)-6,6-dimethyl-4,5,6,7-tetrahydro-1H-indazol-3-yl)-1,2,4-oxadiazol-3-yl)-3-ethyl-6-methylpyridin-2(1H)-one
3-(2,6-Dimethyl-4-(5-(1,6,6-trimethyl-4,5,6,7-tetrahydro-1H-indazol-3-yl)-1,2,4-oxadiazol-3-yl)phenyl)propane-1,2-diol,
N-(2,6-Dimethyl-4-(5-(1,6,6-trimethyl-4,5,6,7-tetrahydro-1H-indazol-3-yl)-1,2,4-oxadiazol-3-yl)phenethyl)-2,2,2-trifluoroethanamine,
2-(2,6-Dimethyl-4-(5-(1,6,6-trimethyl-4,5,6,7-tetrahydro-1H-indazol-3-yl)-1,2,4-oxadiazol-3-yl)phenethylamino)ethanol,
2-(2,6-Dimethyl-4-(5-(1,6,6-trimethyl-4,5,6,7-tetrahydro-1H-indazol-3-yl)-1,2,4-oxadiazol-3-yl)phenethylamino)ethanoic acid,
1-(2,6-Dimethyl-4-(5-(1,6,6-trimethyl-4,5,6,7-tetrahydro-1H-indazol-3-yl)-1,2,4-oxadiazol-3-yl)phenethyl)azetidine-3-carboxylic acid,
3-(2-Methyl-4-(5-(1,6,6-trimethyl-4,5,6,7-tetrahydro-1H-indazol-3-yl)-1,2,4-oxadiazol-3-yl)phenyl)propanoic acid,
4-(2,6-Dimethyl-4-(5-(1,6,6-trimethyl-4,5,6,7-tetrahydro-1H-indazol-3-yl)-1,2,4-oxadiazol-3-yl)phenethyl)morpholine,
3-(4-(5-(6,6-Dimethyl-4,5,6,7-tetrahydro-1H-indazol-3-yl)-1,2,4-oxadiazol-3-yl)-2,6-dimethylphenyl)propane-1,2-diol,
3-(4-(5-(1-Ethyl-6,6-dimethyl-4,5,6,7-tetrahydro-1H-indazol-3-yl)-1,2,4-oxadiazol-3-yl)phenylamido)propanoic acid,
3-(4-(5-(6,6-Dimethyl-1-(pyridin-3-ylmethyl)-4,5,6,7-tetrahydro-1H-indazol-3-yl)-1,2,4-oxadiazol-3-yl)-2,6-dimethylphenyl)propane-1,2-diol,
3-(2-Chloro-4-(5-(1-ethyl-6,6-dimethyl-4,5,6,7-tetrahydro-1H-indazol-3-yl)-1,2,4-oxadiazol-3-yl)phenylsulfonamido)propanoic acid,
3-(4-(5-(6,6-Dimethyl-4,5,6,7-tetrahydrobenzo[d]isoxazol-3-yl)-1,2,4-oxadiazol-3-yl)-2,6-dimethylphenyl)propane-1,2-diol,
3-(1-Ethyl-6,6-dimethyl-4,5,6,7-tetrahydro-1H-indazol-3-yl)-5-(pyridin-4-yl)-1,2,4-oxadiazole,
5-(1-Ethyl-6,6-dimethyl-4,5,6,7-tetrahydro-1H-indazol-3-yl)-3-o-tolyl-1,2,4-oxadiazole,
3-(5-(5-(1-Ethyl-6,6-dimethyl-4,5,6,7-tetrahydro-1H-indazol-3-yl)-1,2,4-oxadiazol-3-yl)-1H-pyrrolo[2,3-b]pyridin-1-yl)propanoic acid,
3-(5-(5-(6,6-Dimethyl-4,5,6,7-tetrahydrobenzo[d]isoxazol-3-yl)-1,2,4-oxadiazol-3-yl)-1H-pyrrolo[2,3-b]pyridin-1-yl)propanoic acid,
1-amino-3-{2,6-dimethyl-4-[5-(1,6,6-trimethyl-4,5,6,7-tetrahydro-1H-indazol-3-yl)-1,2,4-oxadiazol-3-yl]phenyl}propan-2-ol,
N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-2-methylpropan-2-amine,
3-{4-[3-(1-ethyl-6,6-dimethyl-4,5,6,7-tetrahydro-1H-indazol-3-yl)-1,2,4-oxadiazol-5-yl]-3-methylphenyllpropanoic acid,
[2-(4-{5-[6,6-dimethyl-1-(pyridin-3-ylmethyl)-4,5,6,7-tetrahydro-1H-indazol-3-yl]-1,2,4-oxadiazol-3-yl}-2,6-dimethylphenyl)ethyl]amine,
3-(4-{5-[1-(cyclopropylmethyl)-6,6-dimethyl-4,5,6,7-tetrahydro-1H-indazol-3-yl]-1,2,4-oxadiazol-3-yl}-2,6-dimethylphenyl)propanoic acid,
(2-{4-[3-(1-ethyl-6,6-dimethyl-4,5,6,7-tetrahydro-1H-indazol-3-yl)-1,2,4-oxadiazol-5-yl]-3-methylphenyl}ethyl)amine,
N-[2-(4-{5-[6,6-dimethyl-1-(pyridin-3-ylmethyl)-4,5,6,7-tetrahydro-1H-indazol-3-yl]-1,2,4-oxadiazol-3-yl}-2,6-dimethylphenyl)ethyl]glycine,
3-{4-[5-(1-ethyl-6,6-difluoro-4,5,6,7-tetrahydro-1H-indazol-3-yl)-1,2,4-oxadiazol-3-yl]-2,6-dimethylphenyl}propanoic acid,
3-(4-{5-[6,6-dimethyl-1-(pyridin-3-ylmethyl)-4,5,6,7-tetrahydro-1H-indazol-3-yl]-1,2,4-oxadiazol-3-yl}-2,6-dimethylphenyl)propanoic acid,
N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-2-hydroxyacetamide,
3-{4-[5-(1-ethyl-6,6-dimethyl-4,5,6,7-tetrahydro-1H-indazol-3-yl)-1,2,4-oxadiazol-3-yl]-2,6-dimethylphenyl}-N-(methylsulfonyl)propanamide,
3-{4-[5-(1-ethyl-6,6-dimethyl-4,5,6,7-tetrahydro-1H-indazol-3-yl)-1,2,4-oxadiazol-3-yl]-2-methylphenyl}propanoic acid,
3-{4-[5-(1-ethyl-6,6-dimethyl-4,5,6,7-tetrahydro-1H-indazol-3-yl)-1,2,4-oxadiazol-3-yl]-2,6-dimethylphenyl}propane-1,2-diol,
N-(2-{4-[5-(6,6-dimethyl-4,5,6,7-tetrahydro-1H-indazol-3-yl)-1,2,4-oxadiazol-3-yl]-2,6-dimethylphenyl}ethyl)-2,2,2-trifluoroethanamine,
N-({4-[5-(1-ethyl-6,6-dimethyl-4,5,6,7-tetrahydro-1H-indazol-3-yl)-1,2,4-oxadiazol-3-yl]-3-methylphenyl}sulfonyl)-beta-alanine,
N-(3-{4-[5-(1-ethyl-6,6-dmethyl-4,5,6,7-tetrahydro-1H-indazol-3-yl)-1,2,4-oxadiazol-3-yl]-2,6-dimethylphenyl}propyl)methanesulfonamide,
N-(3-{2,6-dimethyl-4-[5-(1,6,6-trimethyl-4,5,6,7-tetrahydro-1H-indazol-3-yl)-1,2,4-oxadiazol-3-yl]phenyl}propanoyl)glycine,
(2-{4-[5-(1-ethyl-6,6-dimethyl-4,5,6,7-tetrahydro-1H-indazol-3-yl)-1,2,4-oxadiazol-3-yl]-2-methylphenyl}ethyl)amine,
N-({4-[5-(1-ethyl-6,6-dimethyl-4,5,6,7-tetrahydro-1H-indazol-3-yl)-1,2,4-oxadiazol-3-yl]-3-methylphenyl}sulfonyl)glycine,
1-ethyl-6,6-dimethyl-3-[3-(2-methyl-4-piperidin-4-ylphenyl)-1,2,4-oxadiazol-5-yl]-4,5,6,7-tetrahydro-1 H-indazole,
(4-{4-[5-(1-ethyl-6,6-dimethyl-4,5,6,7-tetrahydro-1H-indazol-3-yl)-1,2,4-oxadiazol-3-yl]-3-methylphenyl}piperidin-1-yl)acetic acid,
3-(4-{4-[5-(1-ethyl-6,6-dimethyl-4,5,6,7-tetrahydro-1H-indazol-3-yl)-1,2,4-oxadiazol-3-yl]-3-methylphenyl}piperidin-1-yl)propanoic acid,
(2-{4-[3-(1-ethyl-6,6-dimethyl-4,5,6,7-tetrahydro-1H-indazol-3-yl)-1,2,4-oxadiazol-5-yl]-2,6-dimethylphenoxy}ethyl)amine,
3-{4-[5-(1-ethyl-6,6-dimethyl-4,5,6,7-tetrahydro-1H-indazol-3-yl)-1,2,4-oxadiazol-3-yl]-2,5-dimethylphenyl}propanoic acid,
{4-[3-(1-ethyl-6,6-dimethyl-4,5,6,7-tetrahydro-1H-indazol-3-yl)-1,2,4-oxadiazol-5-yl]-2,6-dimethylphenoxy}acetic acid,
3-{4-[3-(1-ethyl-6,6-dimethyl-4,5,6,7-tetrahydro-1H-indazol-3-yl)-1,2,4-oxadiazol-5-yl]-2,6-dimethoxyphenyl}propanoic acid,
3-{2-chloro-4-[3-(1-ethyl-6,6-dimethyl-4,5,6,7-tetrahydro-1H-indazol-3-yl)-1,2,4-oxadiazol-5-yl]-6-methoxyphenyl}propanoic acid,
(2-{2,6-dimethyl-4-[5-(1,6,6-trimethyl-4,5,6,7-tetrahydro-1H-indazol-3-yl)-1,2,4-oxadiazol-3-yl]phenyl}ethyl)[2-(methylsulfonyl)ethyl]amine,
3-{2-ethyl-4-[5-(1-ethyl-6,6-dimethyl-4,5,6,7-tetrahydro-1H-indazol-3-yl)-1,2,4-oxadiazol-3-yl]-6-methylphenyl}propanoic acid,
(2-{3-(trifluoromethyl)-4-[5-(1,6,6-trimethyl-4,5,6,7-tetrahydro-1H-indazol-3-yl)-1,2,4-oxadiazol-3-yl]phenyl}ethyl)amine,
{4-[5-(1-ethyl-6,6-dimethyl-4,5,6,7-tetrahydro-1H-indazol-3-yl)-1,2,4-oxadiazol-3-yl]-2,6-dimethylphenyl}acetic acid,
[3-({3-ethyl-5-[5-(1-ethyl-6,6-dimethyl-4,5,6,7-tetrahydro-1H-indazol-3-yl)-1,2,4-oxadiazol-3-yl]-6-methylpyridin-2-yl}oxy)propyl]amine,
1,6,6-trimethyl-3-[3-(2-methyl-4-piperidin-4-ylphenyl)-1,2,4-oxadiazol-5-yl]-4,5,6,7-tetrahydro-1H-indazole,
2-{4-[5-(1-ethyl-6,6-dimethyl-4,5,6,7-tetrahydro-1H-indazol-3-yl)-1,2,4-oxadiazol-3-yl]-2,6-dimethylphenyl}acetamide,
2-{4-[5-(1-ethyl-6,6-dimethyl-4,5,6,7-tetrahydro-1H-indazol-3-yl)-1,2,4-oxadiazol-3-yl]-2,6-dimethylphenyl}acetamide,
(2-{3-ethyl-5-[5-(1-ethyl-6,6-dimethyl-4,5,6,7-tetrahydro-1H-indazol-3-yl)-1,2,4-oxadiazol-3-yl]-6-methylpyridin-2-yl}ethyl)amine,
(2-{2,6-dimethyl-4-[5-(1,6,6-trimethyl-4,5,6,7-tetrahydro-1H-indazol-3-yl)-1,3,4-thiadiazol-2-yl]phenoxy}ethyl)amine,
{2-[4-[5-(1-ethyl-6,6-dimethyl-4,5,6,7-tetrahydro-1H-indazol-3-yl)-1,2,4-oxadiazol-3-yl]-2-(trifluoromethyl)phenyl]ethyl}amine,
2-{2,6-dimethyl-4-[5-(1,6,6-trimethyl-4,5,6,7-tetrahydro-1H-indazol-3-yl)-1,2,4-oxadiazol-3-yl]phenyl}ethanol,
1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid,
[1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidin-4-yl]acetic acid,
1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)pyrrolidine-3-carboxylic acid,
(3S)-1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)pyrrolidine-3-carboxylic acid,
N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-L-alanine,
N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-2-methylalanine,
N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-D-alanine,
2-((2,6-dimethyl-4-(5-(1,6,6-trimethyl-4,5,6,7-tetrahydro-1H-indazol-3-yl)-1,2,4-oxadiazol-3-yl)phenethyl)(methyl)amino)acetic acid,
(2-{2,6-dimethyl-4-[5-(1,6,6-trimethyl-4,5,6,7-tetrahydro-1H-indazol-3-yl)-1,3,4-oxadiazol-2-yl]phenoxy}ethyl)amine,
{2-[4-[5-(1-ethyl-6,6-dimethyl-4,5,6,7-tetrahydro-1H-indazol-3-yl)-1,2,4-oxadiazol-3-yl]-2-(trifluoromethyl)phenoxy]ethyl}amine,
(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)amine,
(2-{2-(trifluoromethyl)-4-[5-(1,6,6-trimethyl-4,5,6,7-tetrahydro-1H-indazol-3-yl)-1,2,4-oxadiazol-3-yl]phenoxy}ethyl)amine,
(2,2-difluoro-2-{2-methyl-4-[5-(1,6,6-trimethyl-4,5,6,7-tetrahydro-1H-indazol-3-yl)-1,2,4-oxadiazol-3-yl]phenyl}ethyl)amine,
1-{2,6-dimethyl-4-[5-(1,6,6-trimethyl-4,5,6,7-tetrahydro-1H-indazol-3-yl)-1,2,4-oxadiazol-3-yl]phenyl}propan-2-ol,
3-{2,6-dimethyl-4-[5-(1,6,6-trimethyl-4,5,6,7-tetrahydro-1H-indazol-3-yl)-1,2,4-oxadiazol-3-yl]phenyl}propan-1-ol,,
[4-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperazin-1-yl]acetic acid,
1-(2-{4-[5-(1-ethyl-6,6-dimethyl-4,5,6,7-tetrahydro-1H-indazol-3-yl)-1,2,4-oxadiazol-3-yl]-2,6-dimethylphenyl}ethyl)piperidine-4-carboxylic acid,
1-(2-{4-[5-(1-ethyl-6,6-dimethyl-4,5,6,7-tetrahydro-1H-indazol-3-yl)-1,2,4-oxadiazol-3-yl]-2-methylphenyl}ethyl)piperidine-4-carboxylic acid,
1-(2-{4-[3-(1-ethyl-6,6-dimethyl-4,5,6,7-tetrahydro-1H-indazol-3-yl)-1,2,4-oxadiazol-5-yl]-2-methylphenyl}ethyl)piperidine-4-carboxylic acid,
1-{2-[4-[5-(1-ethyl-6,6-dimethyl-4,5,6,7-tetrahydro-1H-indazol-3-yl)-1,2,4-oxadiazol-3-yl]-2-(trifluoromethyl)phenyl]ethyl}piperidine-4-carboxylic acid,
N-{2-[4-[5-(1-ethyl-6,6-dimethyl-4,5,6,7-tetrahydro-1H-indazol-3-yl)-1,2,4-oxadiazol-3-yl]-2-(trifluoromethyl)phenyl]ethyl}glycine,
4-[5-(1,6,6-trimethyl-4,5,6,7-tetrahydro-1H-indazol-3-yl)-1,2,4-oxadiazol-3-yl]benzoic acid,
1-(2-{3-methyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid,
(1-{2-[4-[5-(1-ethyl-6,6-dimethyl-4,5,6,7-tetrahydro-1H-indazol-3-yl)-1,2,4-oxadiazol-3-yl]-2-(trifluoromethyl)phenyl]ethyl}piperidin-4-yl)acetic acid,
2-[(2-{4-[5-(6,6-dimethyl-4,5,6,7-tetrahydro-1,2-benzisoxazol-3-yl)-1,2,4-oxadiazol-3-yl]-2,6-dimethylphenyl}ethyl)amino]ethanol,
N-{2-[(2-{4-[5-(6,6-dimethyl-4,5,6,7-tetrahydro-1,2-benzisoxazol-3-yl)-1,2,4-oxadiazol-3-yl]-2,6-dimethylphenyl}ethyl)amino]ethyl}methane-sulfonamide,
N-(2-{4-[5-(6,6-dimethyl-4,5,6,7-tetrahydro-1,2-benzisoxazol-3-yl)-1,2,4-oxadiazol-3-yl]-2,6-dimethylpheny}ethyl)-2,2,2-trifluoroethanamine,
1-(2-{4-[5-(6,6-dimethyl-4,5,6,7-tetrahydro-1,2-benzisoxazol-3-yl)-1,2,4-oxadiazol-3-yl]-2,6-dimethylphenyl}ethyl)piperidine-4-carboxylic acid,
3-{4-[5-(6,6-dimethyl-4,5,6,7-tetrahydro-1,2-benzisoxazol-3-yl)-1,2,4-oxadiazol-3-yl]-2,6-dimethylphenyl}propane-1,2-diol,
1-(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)piperidine-4-carboxylic acid,
2-(2-(trifluoromethyl)-4-(5-(1,6,6-trimethyl-4,5,6,7-tetrahydro-1H-indazol-3-yl)-1,2,4-oxadiazol-3-yl)phenethylamino)acetic acid,
2-(methyl(2-(trifluoromethyl)-4-(5-(1,6,6-trimethyl-4,5,6,7-tetrahydro-1H-indazol-3-yl)-1,2,4-oxadiazol-3-yl)phenethyl)amino)acetic acid,
1-(2-(trifluoromethyl)-4-(5-(1,6,6-trimethyl-4,5,6,7-tetrahydro-1H-indazol-3-yl)-1,2,4-oxadiazol-3-yl)phenethyl)azetidine-3-carboxylic acid,
2-(1-(2-(trifluoromethyl)-4-(5-(1,6,6-trimethyl-4,5,6,7-tetrahydro-1H-indazol-3-yl)-1,2,4-oxadiazol-3-yl)phenethyl)piperidin-4-yl)acetic acid,
1-(2,6-dimethyl-4-(5-(1,6,6-trimethyl-4,5,6,7-tetrahydro-1H-indazol-3-yl)-4H-1,2,4-triazol-3-yl)phenethyl)piperidine-4-carboxylic acid,
1-(2,6-dimethyl-4-(5-(1,6,6-trimethyl-4,5,6,7-tetrahydro-1H-indazol-3-yl)-1,3,4-oxadiazol-2-yl)phenethyl)piperidine-4-carboxylic acid,
1-(2,6-dimethyl-4-(5-(1,6,6-trimethyl-4,5,6,7-tetrahydro-1H-indazol-3-yl)-1,3,4-thiadiazol-2-yl)phenethyl)piperidine-4-carboxylic acid,
2-(1-(2,6-dimethyl-4-(5-(1,6,6-trimethyl-4,5,6,7-tetrahydro-1H-indazol-3-yl)-1,3,4-thiadiazol-2-yl)phenethyl)piperidin-4-yl)acetic acid,
1-(2-(trifluoromethyl)-4-(5-(1,6,6-trimethyl-4,5,6,7-tetrahydro-1H-indazol-3-yl)-1,3,4-oxadiazol-2-yl)phenethyl)piperidine-4-carboxylic acid,
1-(2-(trifluoromethyl)-4-(5-(1,6,6-trimethyl-4,5,6,7-tetrahydro-1H-indazol-3-yl)-1,3,4-thiadiazol-2-yl)phenethyl)piperidine-4-carboxylic acid,
2-(1-(2-(trifluoromethyl)-4-(5-(1,6,6-trimethyl-4,5,6,7-tetrahydro-1 H-indazol-3-yl)-1,3,4-thiadiazol-2-yl)phenethyl)piperidin-4-yl)acetic acid,
2-(5-(5-(1-ethyl-6,6-dimethyl-4,5,6,7-tetrahydro-1H-indazol-3-yl)-1,2,4-oxadiazol-3-yl)-1 H-pyrrolo[2,3-b]pyridin-1-yl)ethanamine,
5-(1-ethyl-6,6-dimethyl-4,5,6,7-tetrahydro-1H-indazol-3-yl)-3-(1H-pyrrolo[2,3-b]pyridin-5-yl)-1,2,4-oxadiazole,
2-(5-(5-(1,6,6-trimethyl-4,5,6,7-tetrahydro-1H-indazol-3-yl)-1,2,4-oxadiazol-3-yl)-1H-pyrrolo[2,3-b]pyridin-1-yl)ethanamine,
3-(1H-indazol-5-yl)-5-(1,6,6-trimethyl-4,5,6,7-tetrahydro-1H-indazol-3-yl)-1,2,4-oxadiazole,
5-(1-ethyl-6,6-dimethyl-4,5,6,7-tetrahydro-1H-indazol-3-yl)-3-(1H-indol-4-yl)-1,2,4-oxadiazole,
3-(1H-indol-4-yl)-5-(1,6,6-trimethyl-4,5,6,7-tetrahydro-1H-indazol-3-yl)-1,2,4-oxadiazole,
5-(1-ethyl-6,6-dimethyl-4,5,6,7-tetrahydro-1H-indazol-3-yl)-3-(1H-indol-5-yl)-1,2,4-oxadiazole,
3-(1H-benzo[d]imidazol-5-yl)-5-(1-ethyl-6,6-dimethyl-4,5,6,7-tetrahydro-1H-indazol-3-yl)-1,2,4-oxadiazole,
2-(4-(5-(1-ethyl-6,6-dimethyl-4,5,6,7-tetrahydro-1H-indazol-3-yl)-1,2,4-oxadiazol-3-yl)-1 H-indol-1-yl)acetic acid,
2-(5-(5-(1,6,6-trimethyl-4,5,6,7-tetrahydro-1H-indazol-3-yl)-1,2,4-oxadiazol-3-yl)-1H-indol-1-yl)acetic acid,
3-(4-(5-(1-ethyl-6,6-dimethyl-4,5,6,7-tetrahydro-1H-indazol-3-yl)-1,2,4-oxadiazol-3-yl)-1H-indol-1-yl)propanoic acid,
2-(5-(5-(1-ethyl-6,6-dimethyl-4,5,6,7-tetrahydro-1H-indazol-3-yl)-1,2,4-oxadiazol-3-yl)-1H-indol-1-yl)acetic acid,
3-(5-(5-(1-ethyl-6,6-dimethyl-4,5,6,7-tetrahydro-1H-indazol-3-yl)-1,2,4-oxadiazol-3-yl)-1H-indol-1-yl)propanoic acid,
2-(1-(2-(trifluoromethyl)-4-(5-(1,6,6-trimethyl-4,5,6,7-tetrahydro-1H-indazol-3-yl)-1,3,4-oxadiazol-2-yl)phenethyl)piperidin-4-yl)acetic acid,
2-(2-(trifluoromethyl)-4-(5-(1,6,6-trimethyl-4,5,6,7-tetrahydro-1H-indazol-3-yl)-1,3,4-thiadiazol-2-yl)phenethylamino)acetic acid,
1-(2-(trifluoromethyl)-4-(5-(1,6,6-trimethyl-4,5,6,7-tetrahydro-1H-indazol-3-yl)-1,3,4-thiadiazol-2-yl)phenethyl)azetidine-3-carboxylic acid,
1-(2-(trifluoromethyl)-4-(3-(1,6,6-trimethyl-4,5,6,7-tetrahydro-1H-indazol-3-yl)-1,2,4-oxadiazol-5-yl)phenethyl)piperidine-4-carboxylic acid
2-(1-(2-(trifluoromethyl)-4-(3-(1,6,6-trimethyl-4,5,6,7-tetrahydro-1H-indazol-3-yl)-1,2,4-oxadiazol-5-yl)phenethyl)piperidin-4-yl)acetic acid
3-(3-(trifluoromethyl)-4-(5-(1,6,6-trimethyl-4,5,6,7-tetrahydro-1H-indazol-3-yl)-1,2,4-oxadiazol-3-yl)phenyl)propanoic acid,
3-(4-(5-(1-ethyl-6,6-dimethyl-4,5,6,7-tetrahydro-1H-indazol-3-yl)-1,2,4-oxadiazol-3-yl)-2-(trifluoromethyl)phenyl)propanoic acid, and
3-(2-(trifluoromethyl)-4-(5-(1,6,6-trimethyl-4,5,6,7-tetrahydro-1H-indazol-3-yl)-1,2,4-oxadiazol-3-yl)phenyl)propanoic acid
or a pharmaceutically acceptable salt, or solvate, or N-oxide, or stereoisomer or deuterated derivate thereof.

22. The S1P1 receptor agonist according to claim 21 which is 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid or a pharmaceutically acceptable salt, or solvate, or N-oxide, or stereoisomer or deuterated derivate thereof.

23. The S1P1 receptor agonist according to claim 22, wherein the pharmaceutically acceptable salt is hydrochloride.

24. The S1P1 receptor agonist according to claim 21 which is 1-(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)piperidine-4-carboxylic acid or a pharmaceutically acceptable salt, or solvate, or N-oxide, or stereoisomer or deuterated derivate thereof.

25. The S1P1 receptor agonist according to claim 24, wherein the pharmaceutically acceptable salt is hydrochloride.

26. A combination for use in the treatment of Crohn's disease, which combination comprising a S1P1 receptor agonist of formula (I) as defined in any one of claims 1-25 and another active compound seleted from:
(a) Anti-TNF alpha agents such as infliximab and adalimumab,
(b) immunosuppressants such as azathioprine and 6-mercapthopurine,
(c) anti-inflammatory agents such as sulphasalazine,
(d) anti-alpha 4 integrin antibodies, such as Natalizumab (Tysabri),
(e) alpha 4 integrin antagonists such as R-1295 , TBC-4746, CDP-323, ELND-002, Firategrast and TMC-2003,
(f) , dyhydrofolate reductase inhibitors, such as Methotrexate or CH-1504,
(g) glucocorticoids such as prednisone or methylprednisolone,
(h) DHODH inhibitors such as Teriflunomide and compounds disclosed in WO 2008/077639 and WO2009021696.
(i) cannabinoid receptor agonists such as Sativex, and
(j) chemokine CCR1 antagonists such as MLN-3897 or PS-031291.

27. Use of a compound of formula (I) as defined in any one of claims 1-25, or a combination as defined in claim 26 in the manufacture of a medicament for use in the treatment of Crohn's disease.

28. A product comprising (a) a compound of formula (I) as defined in any one of claims 1 to 25 and (b) an active compound as defined in claim 26, as a combined preparation for simultaneous, separate or sequential use in the treatment of Crohn's disease.

29. A kit of parts comprising (a) a compound of formula (I) as defined in any one of claims 1 to 25 together with instructions for simultaneous, separate or sequential use in combination with (b) an active compound as defined in claim 26 for the treatment of Crohn's disease.

30. A package comprising (a) a compound of formula (I) as defined in any one of claims 1 to 25 and (b) an active compound as defined in claim 26 for simultaneous, separate or sequential use in the treatment of a Crohn's disease.
